# EUROPEAN PATENT APPLICATION

(11) **EP 3 985 028 A1**
(43) Date of publication of application: **20.04.2022**
(21) Application number: 21202957.3
(22) Date of filing: 06.02.2014
(51) Int. Cl.: C07K 16/32, C07K 16/22, C07K 16/28, C07K 14/725, A61K 39/00, A61K 48/00, A01N 63/00, C12N 5/0783

(54) **MODIFIED T LYMPHOCYTES HAVING IMPROVED SPECIFICITY**

(30) Priority: 06.02.2013 US 201361761548 P
(62) Divisional of application: 19182025.7
(71) Applicant: Celgene Corporation, Summit, NJ 07901 (US)
(72) Inventor: LIANG, Bitao, Closter, 07624 (US); ABBOT, Stewart, San Diego, 92130 (US); LIU, Wei, Bridgewater, 08807 (US)
(74) Representative: Jones Day

(57) **Abstract**

Provided herein is a modified T lymphocyte comprising a first polypeptide comprising a first extracellular antigen binding domain that binds a first antigen, and a first intracellular signaling domain, wherein said first polypeptide does not comprise a co-stimulatory domain; and a second polypeptide comprising a second extracellular antigen binding domain binding a second antigen, or a receptor that binds said second antigen; and a second intracellular signaling domain, wherein said second polypeptide comprises one or more co-stimulatory domains, wherein said modified lymphocyte becomes maximally cytotoxic only when said first intracellular signaling domain and said second intracellular signaling domain are both activated by said first antigen and said second antigen, respectively. Also provided herein is a modified T lymphocyte of the present invention for use in a method of treating a tumor or cancer in an individual.

## Description

This application claims priority to U.S. Provisional Patent Application No. 61/761,548, filed February 6, 2013, the disclosure of which is incorporated herein by reference in its entirety.

### 1. FIELD

The disclosure herein relates to the field of immunology, and more specifically, to the modification of T lymphocytes or other immune cells.

### 2. BACKGROUND

Cells of the immune system such as T lymphocytes (also referred to as T cells) recognize and interact with specific antigens through receptors or receptor complexes which, upon recognition or an interaction with such antigens, cause activation of the cell. An example of such a receptor is the antigen-specific T lymphocyte receptor complex (TCR/CD3), a complex of eight proteins. The T cell receptor (TCR) is expressed on the surface of T lymphocytes. One component, CD3, which has an invariant structure, is responsible for intracellular signaling following occupancy of the TCR by ligand. The T lymphocyte receptor for antigen-CD3 complex (TCR/CD3) recognizes antigenic peptides that are presented to it by the proteins of the major histocompatibility complex (MHC). Complexes of MHC and peptide are expressed on the surface of antigen presenting cells and other T lymphocyte targets. Stimulation of the TCR/CD3 complex results in activation of the T lymphocyte and a consequent antigen-specific immune response. The TCR/CD3 complex plays a central role in the effector function and regulation of the immune system.

T lymphocytes require a second, co-stimulatory signal to become fully active. Without such a signal, T lymphocytes are either non-responsive to antigen binding to the TCR, or become anergic. Such a co-stimulatory signal, for example, is provided by CD28, a T lymphocyte protein, which interacts with CD80 and CD86 on antigen-producing cells. ICOS (Inducible COStimulator), another T lymphocyte protein, provides a co-stimulatory signal when bound to ICOS ligand.

The essential antigen-binding, signaling and stimulatory functions of the TCR complex have been reduced by genetic recombination methods to a single polypeptide chain, generally referred to as a Chimeric Antigen Receptor (CAR). *See, e.g.,* Eshhar, U.S. Patent No. 7,741,465; Eshhar, U.S. Patent Application Publication No. 2012/0093842. T lymphocytes bearing such CARs are generally referred to as CAR-T lymphocytes. However, while such CARs effectively target T lymphocytes to specific tumor-associated antigen or tumor-specific antigen, and can effectively target T lymphocytes to kill tumor cells expressing such antigens, such a design suffers the serious side effect of directing T lymphocytes to kill normal, healthy cells that also express such antigens. As such, use of such CAR-T lymphocytes is generally restricted to tumors expressing an antigen not expressed by any other cell in the body, a relatively rare circumstance.

Described herein are modified T lymphocytes comprising chimeric receptors that overcome this shortcoming of current CAR design.

### 3. SUMMARY

In a first aspect, provided herein are modified lymphocytes, e.g., modified T lymphocytes that comprise at least two different polypeptides, e.g., chimeric receptors, in which the immune signal derived from binding of a first polypeptide, e.g., chimeric receptor, to a first antigen is separated from a costimulatory signal produced by a second polypeptide, e.g., chimeric receptors, and wherein the costimulatory signal is dependent on antigen binding of a second antigen by the second chimeric receptors. As used throughout herein, "first polypeptide" indicates the polypeptide generating the primary antigen-binding immune signal, and the "second polypeptide" is the polypeptide generating the co-stimulatory immune signal. In certain embodiments, the two polypeptides (e.g., chimeric receptors) are introduced into the modified T lymphocytes using a single CAR construct, with the polypeptides are separated by a cleavable sequence (T2A or P2A) that allows for the expression of two, discrete polypeptides (at essentially equal amounts) from a single ORF.

In one embodiment, provided herein is a modified T lymphocyte (CAR-T lymphocyte) comprising a first polypeptide comprising a first extracellular antigen binding domain that binds a first antigen, and a first intracellular signaling domain, wherein said first polypeptide does not comprise a co-stimulatory domain; and a second polypeptide comprising a second extracellular antigen binding domain binding a second antigen, or a receptor that binds said second antigen; and a second intracellular signaling domain; wherein said modified lymphocyte becomes maximally cytotoxic only when said first signaling domain and said second signaling domain are both activated by said first antigen and said second antigen, respectively. In a specific embodiment, binding of said first antigen to said first antigen binding domain without binding of said second antigen to said second binding domain, or binding of said second antigen to said second antigen binding domain without binding of first second antigen to said first binding domain, induces anergy of said modified T lymphocyte, or non-responsiveness of said T-lymphocyte to said first antigen or said second antigen.

In another specific embodiment, said first antigen binding domain and said second antigen binding domain are independently an antigen-binding portion of a receptor, an antigen-binding portion of an antibody, or other peptide-based macromolecular antigen binding agent. In certain specific embodiments, either or both of said first antigen binding domain or said second antigen binding domain are scFv antibody fragments. In specific embodiments, either or both of said first polypeptide or said second polypeptide additionally comprise a transmembrane domain. In other specific embodiments, said first polypeptide or said second polypeptide comprises a T cell survival motif. In a specific embodiment, the T cell survival motif is a CD28 T cell survival motif. In other specific embodiments, said T cell survival motif is an intracellular signaling domain of IL-7 receptor (IL-7R), an intracellular signaling domain of IL-12 receptor, an intracellular signaling domain of IL-15 receptor, an intracellular signaling domain of IL-21 receptor, or an intracellular signaling domain of transforming growth factor ß (TGFß) receptor. In another more specific embodiment, said first polypeptide or said second polypeptide comprises a portion of a CD28 molecule that comprises a T cell survival motif. In a more specific embodiment, said first polypeptide or said second polypeptide comprises a CD28 molecule that comprises a T cell survival motif. In certain specific embodiments, said first intracellular signaling domain comprises a polypeptide sequence comprising an immunoreceptor tyrosine-based activation motif (ITAM). In a more specific embodiment, said polypeptide sequence is a CD3ζ signaling domain.

In certain specific embodiments, said first antigen is an antigen on a tumor cell. In a more specific embodiment, said tumor cell is a cell in a solid tumor. In another more specific embodiment, said tumor cell is a blood cancer cell. In another specific embodiment, said antigen is a tumor-associated antigen or a tumor-specific antigen. In more specific embodiments, said tumor-associated antigen or tumor-specific antigen is Her2, prostate stem cell antigen (PSCA), PSMA (prostate-specific membrane antigen), B cell maturation antigen (BCMA), alpha-fetoprotein (AFP), carcinoembryonic antigen (CEA), cancer antigen-125 (CA-125), CA19-9, calretinin, MUC-1, epithelial membrane protein (EMA), epithelial tumor antigen (ETA), tyrosinase, melanoma-associated antigen (MAGE), CD34, CD45, CD99, CD117, chromogranin, cytokeratin, desmin, glial fibrillary acidic protein (GFAP), gross cystic disease fluid protein (GCDFP-15), HMB-45 antigen, protein melan-A (melanoma antigen recognized by T lymphocytes; MART-1), myo-D1, muscle-specific actin (MSA), neurofilament, neuron-specific enolase (NSE), placental alkaline phosphatase, synaptophysin, thyroglobulin, thyroid transcription factor-1, the dimeric form of the pyruvate kinase isoenzyme type M2 (tumor M2-PK), CD19, CD22, CD27, CD30, CD70, GD2 (ganglioside G2), EphA2, CSPG4, CD138, FAP (Fibroblast Activation Protein), CD171, kappa, lambda, 5T4, aᵥβ₆ integrin, B7-H3, B7-H6, CAIX, CD19, CD20, CD22, CD30, CD33, CD44, CD44v6, CD44v7/8, CD70, CD123, EGFR, EGP2, EGP40, EpCAM, fetal AchR, FRα, GD3, HLA-A1+MAGE1, HLA-A1+NY-ESO-1, IL-11Rα, IL-13Rα2, Lewis-Y, Muc16, NCAM, NKG2D Ligands, NY-ESO-1, PRAME, ROR1, Survivin, TAG72, TEMs, VEGFR2, EGFRvIII (epidermal growth factor variant III), sperm protein 17 (Sp17), mesothelin, PAP (prostatic acid phosphatase), prostein, TARP (T cell receptor gamma alternate reading frame protein), Trp-p8, STEAP1 (six-transmembrane epithelial antigen of the prostate 1), an abnormal ras protein, or an abnormal p53 protein. In a specific embodiment, said first antigen is PSCA. In another specific embodiment, said first antigen is PSMA. In another specific embodiment, said first antigen is BCMA. In another specific embodiment, when said first antigen binding domain is specific for HER2, the antigen binding domain of the second polypeptide of the modified T cell is not specific for MUC-1.

In another specific embodiment, said first antigen is integrin αvβ3 (CD61), galactin, K-Ras (V-Ki-ras2 Kirsten rat sarcoma viral oncogene) or Ral-B.

In another specific embodiment, said second antigen is a growth factor, cytokine, or interleukin. In a particular embodiment, the second antigen is a molecule, e.g., a growth factor, cytokine, or interleukin associated with angiogenesis or vasculogenesis. In more specific embodiments, said second antigen is vascular endothelial growth factor (VEGF), basic fibroblast growth factor (bFGF), platelet-derived growth factor (PDGF), hepatocyte growth factor (HGF), insulin-like growth factor (IGF), or interleukin-8 (IL-8). Accordingly, said second antigen binding domain can be, e.g., an antibody (or fragment thereof, e.g., scFv) specific to VEGF, bFGF, PDGF, HGF, IGF, or IL-8; or a receptor for VEGF, bFGF, PDGF, HGF, IGF, or IL-8. In a specific embodiment, said second antigen binding domain is a receptor for VEGF, bFGF, PDGF, HGF, IGF, TGF-beta, IL4, IL-10, IL13, or IL-8. In another specific embodiment, said second antigen binding domain is a receptor for VEGF, wherein said receptor for VEGF is VEGFR, e.g., VEGFR1, VEGFR2, or VEGFR3.

In another specific embodiment, signal transduction activation provided by said second antigen is non-antigenic, but is associated with hypoxia. In more specific embodiments, said stimulus is induced by activation of hypoxia-inducible factor-1α (HIF-1α), HIF-1β, HIF-2α, HIF-2β, HIF-3α, or HIF-3β.

In another specific embodiment, said second antigen is an interleukin.

In another specific embodiment, said second antigen is a damage associated molecular pattern molecule (DAMP; also known as an alarmin). In more specific embodiments, said DAMP is a heat shock protein, chromatin-associated protein high mobility group box 1 (HMGB1), S100A8 (also known as MRP8, or calgranulin A), S100A9 (also known as MRP14, or calgranulin B), serum amyloid A (SAA), deoxyribonucleic acid, adenosine triphosphate, uric acid, or heparin sulfate.

In certain specific embodiments, said second antigen is an antigen on an antibody that binds to an antigen presented by a tumor cell.

In a specific embodiment, provided herein is a modified T lymphocyte (CAR-T lymphocyte) comprising a first polypeptide comprising a first extracellular antigen binding domain that binds a first antigen, and a first intracellular signaling domain, wherein said first polypeptide does not comprise a co-stimulatory domain; and a second polypeptide comprising a second extracellular antigen binding domain binding that binds VEGF, and a second intracellular signaling domain (costimulatory domain); wherein said modified lymphocyte becomes maximally cytotoxic when said first signaling domain and said second signaling domain are both activated by said first antigen and said second antigen, respectively. In a specific embodiment, said first antigen is PSCA, PSMA, or BCMA. In another specific embodiment, said first extracellular antigen binding domain comprises an antibody or fragment thereof (e.g., scFv), e.g., an antibody or fragment thereof specific to PSCA, PSMA, or BCMA. In another specific embodiment, said antigen binding domain binding that binds VEGF is a receptor for VEGF, i.e., VEGFR. In another specific embodiment, said VEGFR is VEGFR1, VEGFR2, or VEGFR3. In another specific embodiment, said VEGFR is VEGFR2.

In a specific embodiment of any of the embodiments herein, said second polypeptide comprises one or more co-stimulatory domains. In specific embodiments, said one or more co-stimulatory domains comprises one or more of a co-stimulatory CD27 polypeptide sequence, a co-stimulatory CD28 polypeptide sequence, a co-stimulatory OX40 (CD134) polypeptide sequence, a co-stimulatory 4-1BB (CD137) polypeptide sequence, TILR2, TILR4, TILR7, TILR9, Fc receptor gamma chain, Fc receptor ε chain, or a co-stimulatory inducible T-cell costimulatory (ICOS) polypeptide sequence.

In a specific embodiment of the modified T lymphocytes provided herein, said first polypeptide comprises an extracellular tumor antigen-binding domain and a CD3ζ signaling domain, and wherein said second polypeptide comprises an antigen-binding domain wherein said antigen is an angiogenic or vasculogenic factor, and one or more co-stimulatory molecule signaling domains. Said angiogenic factor can be, e.g., VEGF. Said one or more co-stimulatory molecule signaling motifs can comprise, e.g., co-stimulatory signaling domains from each of CD27, CD28, OX40, ICOS, and 4-1BB. In a more specific embodiment, said first polypeptide comprises an extracellular tumor antigen-binding domain and a CD3ζ signaling domain, and wherein said second polypeptide comprises an antigen-binding domain wherein said antigen is VEGF, and co-stimulatory signaling domains from each of CD27, CD28, OX40, ICOS, and 4-1BB. In a more specific embodiment, said first polypeptide or said second polypeptide comprises a T cell survival motif. In more specific embodiments, said T cell survival motif is, or is derived from, an intracellular signaling domain of IL-7 receptor (IL-7R), an intracellular signaling domain of IL-12 receptor, an intracellular signaling domain of IL-15 receptor, an intracellular signaling domain of IL-21 receptor, or an intracellular signaling domain of transforming growth factor ß (TGFß) receptor. In a more specific embodiment of said modified T lymphocyte, therefore, said first polypeptide comprises an extracellular tumor antigen-binding domain and a CD3ζ signaling domain, and wherein said second polypeptide comprises an antigen-binding domain wherein said antigen is VEGF, an IL-7 receptor intracellular T cell survival motif, and co-stimulatory signaling domains from each of CD27, CD28, OX40, ICOS, and 4-1BB.

In another specific embodiment of the modified T lymphocyte, said first antigen is a tumor-specific antigen or a tumor-associated antigen, and said first intracellular signaling domain comprises a CD3ζ signaling domain; and wherein said second polypeptide comprises an antigen-binding domain that binds said second antigen, and co-stimulatory signaling domains from each of CD27, CD28, OX40, ICOS, and 4-1BB. In a more specific embodiment, said second polypeptide further comprises an intracellular T cell survival motif, e.g., a T cell survival motif that is, or is derived from, an intracellular signaling domain of IL-7 receptor (IL-7R), an intracellular signaling domain of IL-12 receptor, an intracellular signaling domain of IL-15 receptor, an intracellular signaling domain of IL-21 receptor, or an intracellular signaling domain of transforming growth factor ß (TGFß) receptor.

In a specific embodiment of any of the modified T lymphocytes provided herein, said second antigen is VEGF or IL-4.

In certain embodiments, only said first antigen binding domain or said second antigen binding domain binds to a tumor-associated antigen or a tumor-specific antigen, and the other of said first antigen binding domain or said second antigen binding domain binds to an antigen that is not a tumor-specific antigen or a tumor-associated antigen. In such embodiments, only one of the first or second stimulatory signals is generated by a tumor-specific antigen or tumor-associated antigen; the other of the first or second stimulatory signals is generated by another type of antigen, e.g., an antigen (e.g., protein or other biomolecule) associated with the tumor environment).

Also provided herein, in certain embodiments, are modified T lymphocytes that comprise the first and second polypeptides, and one or more additional polypeptides, e.g., one or more additional polypeptides that comprise an antigen-binding domain and a signaling domain. In specific embodiments, only one of said polypeptides (e.g., only one of said first polypeptide, said second polypeptide, or said one or more additional polypeptides) comprises an antigen binding domain that binds to a tumor-associated antigen or a tumor-specific antigen; each of the remainder of said polypeptides comprises an antigen binding domain that binds to an antigen that is not a tumor-associated antigen or a tumor-specific antigen. In other specific embodiments, two or more of said first polypeptide, said second polypeptides, and said one or more additional polypeptides comprise antigen binding domains that bind to one or more tumor-associated antigens or tumor-specific antigens, wherein at least one of said polypeptides comprises an antigen binding domain that does not bind to a tumor-associated antigen or a tumor-specific antigen.

In another aspect, provided herein is a modified T lymphocyte comprising a first polypeptide comprising a first extracellular antigen binding domain that binds a first antigen, and a first intracellular signaling domain; and a second polypeptide comprising a second extracellular antigen binding domain binding a second antigen, or a receptor that binds said second antigen; and a second intracellular signaling domain, wherein said second polypeptide does not comprise a co-stimulatory domain; wherein said modified lymphocyte becomes maximally cytotoxic only when said first signaling domain and said second signaling domain are both activated by said first antigen and said second antigen, respectively. In a specific embodiment, binding of said first antigen to said first antigen binding domain without binding of said second antigen to said second binding domain, or binding of said second antigen to said second antigen binding domain without binding of first second antigen to said first binding domain induces anergy of said modified T lymphocyte, or non-responsiveness of said modified T lymphocyte to said first antigen. In a specific embodiment, said first antigen-binding domain and said antigen-binding domain are independently an antigen-binding portion of a receptor or an antigen-binding portion of an antibody. In another specific embodiment, either or both of said first antigen binding domain or said second antigen binding domain are scFv antibody fragments. In specific embodiments, said first polypeptide and/or said second polypeptide additionally comprises a transmembrane domain. In a more specific embodiment, said first polypeptide or said second polypeptide comprises a T cell survival motif, *e.g*.., any of the T cell survival motifs described herein.

In another specific embodiment, said first antigen is an antigen on a tumor cell, e.g., a cell in a solid tumor or a blood cancer cell. In a specific embodiment, said first antigen is a tumor-associated antigen or a tumor-specific antigen, e.g., Her2, prostate stem cell antigen (PSCA), PSMA (prostate-specific membrane antigen), B cell maturation antigen (BCMA), alpha-fetoprotein (AFP), carcinoembryonic antigen (CEA), cancer antigen-125 (CA-125), CA19-9, calretinin, MUC-1, epithelial membrane protein (EMA), epithelial tumor antigen (ETA), tyrosinase, melanoma-associated antigen (MAGE), CD34, CD45, CD99, CD117, chromogranin, cytokeratin, desmin, glial fibrillary acidic protein (GFAP), gross cystic disease fluid protein (GCDFP-15), HMB-45 antigen, protein melan-A (melanoma antigen recognized by T lymphocytes; MART-1), myo-D1, muscle-specific actin (MSA), neurofilament, neuron-specific enolase (NSE), placental alkaline phosphatase, synaptophysin, thyroglobulin, thyroid transcription factor-1, the dimeric form of the pyruvate kinase isoenzyme type M2 (tumor M2-PK), an abnormal ras protein, or an abnormal p53 protein. In certain embodiments, the tumor-associated antigen is CD19, CD22, CD27, CD30, CD70, GD2 (ganglioside G2), EGFRvIII (epidermal growth factor variant III), sperm protein 17 (Sp17), mesothelin, PAP (prostatic acid phosphatase), prostein, TARP (T cell receptor gamma alternate reading frame protein), Trp-p8, or STEAP1 (six-transmembrane epithelial antigen of the prostate 1).

In another specific embodiment, said first antigen is integrin αvβ3 (CD61), galactin, K-Ras (V-Ki-ras2 Kirsten rat sarcoma viral oncogene) or Ral-B.

In certain specific embodiments, said second intracellular signaling domain comprises a polypeptide sequence comprising an immunoreceptor tyrosine-based activation motif (ITAM), e.g., a CD3ζ signaling domain. In a specific embodiment, said second antigen is a growth factor, cytokine, or interleukin. In another specific embodiment, said second antigen is a growth factor, cytokine, or interleukin associated with angiogenesis or vasculogenesis, e.g., VEGF, bFGF, PDGF, HGF, IGF, or IL-8. In other more specific embodiments, signal transduction by said second chimeric receptor is induced by activation of a hypoxia-associated factor, e.g., HIF-1α, HIF-1β, HIF-2α, HIF-2β, HIF-3α, or HIF-3β. In other specific embodiments, said second antigen is an interleukin. In other specific embodiments, said second antigen is a DAMP, e.g., a heat shock protein, HMGB1, S100A8, S100A9, SAA, DNA, ATP, uric acid, or heparin sulfate. In other specific embodiments, said second antigen is an administered peptide, e.g., an antibody or a synthetic polypeptide. In other specific embodiments, said second antigen is an antigen on an antibody that binds to an antigen presented by a tumor cell. In certain specific embodiments, said first polypeptide and or said second polypeptide comprises one or more co-stimulatory domains, e.g., one or more of a co-stimulatory CD27 polypeptide sequence, a co-stimulatory CD28 polypeptide sequence, a co-stimulatory OX40 (CD134) polypeptide sequence, a co-stimulatory 4-1BB (CD137) polypeptide sequence, TILR2, TILR4, TILR7, TILR9, Fc receptor gamma chain, Fc receptor ε chain, or a co-stimulatory inducible T-cell co-stimulatory (ICOS) polypeptide sequence. In any of the above embodiments, in a specific embodiment, said first polypeptide or said second polypeptide comprises a T cell survival motif, e.g., said T cell survival motif is, or is derived from, an intracellular signaling domain of IL-7 receptor (IL-7R), an intracellular signaling domain of IL-12 receptor, an intracellular signaling domain of IL-15 receptor, an intracellular signaling domain of IL-21 receptor, or an intracellular signaling domain of transforming growth factor ß (TGFß) receptor.

In another aspect, provided herein is a method of treating an individual having a disease or disorder, wherein the disease or disorder is characterized, or is characterizable, by a first antigen, and is associated with a second antigen. In one embodiment, said first antigen is a tumor-associated antigen or a tumor-specific antigen.

### 3.1 Brief Description of the Drawings

FIG. 1: A schematic representation of four CARs. SP: signal peptide; EC: extracellular; TM: transmembrane; IC: intracellular.

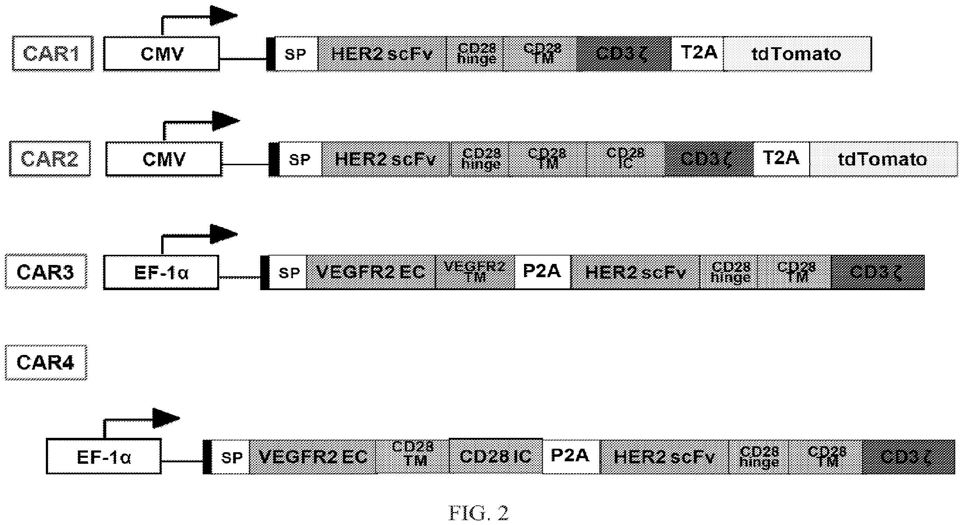

FIG. 2: A schematic representation of four CARs. SP: signal peptide; EC: extracellular; TM: transmembrane; IC: intracellular.

### 4. DETAILED DESCRIPTION

Provided herein are genetically modified immune system cells, e.g., T lymphocytes (T lymphocytes) that are directed to cells displaying a desired antigen, and which are more specific to such cells than existing T lymphocyte-based therapeutics. Generally, existing modified T lymphocytes are modified to express a polypeptide known as a Chimeric Antigen Receptor, or CAR. See, e.g., Eshhar, U.S. Patent No. 7,741,465. T lymphocytes expressing CARs are known as CAR-T lymphocytes. The general structure of a CAR comprises a single polypeptide chain that includes an extracellular portion and an intracellular portion; a transmembrane portion is optionally added to anchor the CAR in the T lymphocyte's cell membrane. The extracellular portion includes a domain or motif that is able to bind an antigen of interest, e.g., an antigen on a cell, for example, a tumor-specific antigen or tumor-associated antigen. The intracellular portion includes a domain or motif that is able to transmit a primary antigen-binding signal that is necessary for the activation of a T lymphocyte in response to the antigen's binding to the CAR's extracellular portion. Typically, this domain or motif comprises, or is, an ITAM (immunoreceptor tyrosine-based activation motif). ITAM-containing polypeptides suitable for CARs include, for example, the zeta CD3 chain (CD3ζ) or ITAM-containing portions thereof. Given that T lymphocytes require a second, co-stimulatory signal for full activation, in more recent iterations of CAR design, the intracellular portion further comprises one or more co-stimulatory motifs, typically a co-stimulatory portion of CD27, CD28 or CD137 (4-1BB). Such a design allows for a T lymphocyte expressing the CAR to become fully activated upon binding of the antigen of interest to the extracellular domain of the CAR, enabling the CAR-T lymphocyte to kill the antigen-bearing cell.

While such modified T lymphocytes can be highly effective at killing undesirable cells bearing a particular antigen, they are also effective at killing normal cells that express low, but detectable, amounts of such antigen. Such off-tumor activity of modified T lymphocytes can result in severe damage to a recipient's normal tissues, and even death. For example, a metastatic colon cancer patient, administered 10¹⁰ CAR-T lymphocytes directed to *ERBB2-*overexpressing tumors, experienced pulmonary distress within 15 minutes after administration, and subsequently died of multiple organ failure and hemorrhage. See Morgan et al., "Case Report of a Serious Adverse Event Following the Administration of T lymphocytes Transfected with a Chimeric Antigen Receptor Recognizing ERBB2," Molecular Therapy 18(4):843-851 (2010). Such deleterious off-tumor effects severely limit the applicability of single-chain CARs and T lymphocytes expressing them.

Such off-tumor effects mediated by CARs would, however, be reduced or eliminated by separating primary antigen-binding signal transduction from co-stimulation, so that antigen binding, alone, is not sufficient to activate the modified T lymphocytes, e.g., wherein both primary and secondary signals would be expected to be uniquely apparent within tumor-bearing, but not normal, tissues. This separation, as disclosed herein, is accomplished through the use of T lymphocytes modified to express a co-stimulatory polypeptide, e.g., chimeric receptor, or modified to express two or more artificial polypeptides, e.g., chimeric receptors, at least one of which comprises a primary antigen-binding signal transduction domain and does not also comprise a co-stimulatory motif, and at least one of which comprises a co-stimulatory domain or motif, but not a primary antigen-binding signal transduction domain, wherein the two or more chimeric receptors do not bind to the same antigen. Non-binding of the co-stimulatory polypeptide in addition to the primary signal-transducing polypeptide (whether the native TCR or an artificial polypeptide) results in non-responsiveness, anergy, or apoptosis of the modified T lymphocytes.

### 4.1. Bispecific Modified T Lymphocytes

### 4.1.1. First Configuration

In certain embodiments, provided herein are modified T lymphocytes that comprise a single artificial co-stimulatory polypeptide, e.g., chimeric receptor, which provides a co-stimulatory signal in response to a particular antigen; the primary antigen-binding signal, however, is transmitted through the native T cell receptor proteins. This single polypeptide comprises, e.g., an antigen binding domain that binds to a first antigen, and one or more co-stimulatory domains, but lacks a primary antigen binding signal-generating domain, such as ITAM or CD3ζ. The modified T cells, in this configuration, rely on the native T cell receptor and CD3ζ signaling protein for primary antigen binding signaling. The co-stimulatory polypeptide provides a co-stimulatory signal that enhances the T lymphocyte's response to the particular antigen. In certain embodiments, the T cell natively recognizes a first antigen, e.g., a tumor-specific antigen (TSA) or tumor-associated antigen (TAA), and the artificial polypeptide (e.g., chimeric receptor), and the co-stimulatory polypeptide's antigen binding domain also binds said first antigen. In other embodiments, the T cell natively recognizes a first antigen, e.g., a tumor-specific antigen (TSA) or tumor-associated antigen (TAA), and the artificial polypeptide (e.g., chimeric receptor), and the co-stimulatory polypeptide's antigen binding domain binds a second, different antigen, e.g., a different TSA or TAA. In other embodiments, the T cell natively recognizes a first antigen, e.g., a tumor-specific antigen (TSA) or tumor-associated antigen (TAA), and the artificial polypeptide (e.g., chimeric receptor), and the co-stimulatory polypeptide's antigen binding domain binds a second antigen that is not a TSA or TAA.

The antigen binding portion of the artificial polypeptide (e.g., chimeric receptor) can be any polypeptide domain, motif or sequence that binds to an antigen. In certain embodiments, the antigen binding domain is an antigen binding portion of a receptor or an antigen-binding portion of an antibody. For example, the antigen-binding domain can be receptors or an antigen binding portion thereof, e.g., a receptor for a ligand produced by a tumor cell, an antibody, antibody chain, or an antigen binding portion thereof, an Fc domain, a glycophosphatidylinositol anchor domain, or the like. In certain embodiments, therefore, the antigen binding is an scFv antibody fragment. In certain other embodiments, the antigen-binding domain is another form of peptide-based macromolecular antigen binding agent, e.g., phage display protein. In certain other embodiments, the antigen binding domain does not bind the antigen directly, but binds to a modified protein that binds the antigen. In a specific embodiment, for example, the antigen binding domain comprises a ligand, e.g., biotin, that binds to a ligand, e.g., avidin, on an antigen-binding polypeptide or macromolecule. In various embodiments, antigen binding by the antigen binding domain can be restricted to antigen presentation in association with major histocompatibility complexes (MHC), or can be MHC-unrestricted.

In certain embodiments, the one or more co-stimulatory domains within the artificial polypeptide (chimeric receptor) comprises one or more of a co-stimulatory CD27 polypeptide sequence, a co-stimulatory CD28 polypeptide sequence, a co-stimulatory OX40 (CD134) polypeptide sequence, a co-stimulatory 4-1BB (CD137) polypeptide sequence, or a co-stimulatory inducible T-cell costimulatory (ICOS) polypeptide sequence.

The first antigen may be any antigen of interest, *e.g.*, an antigen that is expressed on the surface of a cell. In preferred embodiments, said first antigen is an antigen on a tumor cell, e.g., a TAA or TSA. The tumor cell can be a cell, e.g., of a solid tumor or a blood cancer. In certain specific embodiments, said antigen is a tumor-associated antigen or a tumor-specific antigen, e.g., Her2, prostate stem cell antigen (PSCA), PSMA (prostate-specific membrane antigen), B cell maturation antigen (BCMA), ERK5, alpha-fetoprotein (AFP), carcinoembryonic antigen (CEA), cancer antigen-125 (CA-125), CA19-9, calretinin, MUC-1, epithelial membrane protein (EMA), epithelial tumor antigen (ETA), tyrosinase, melanoma-associated antigen (MAGE), CD34, CD45, CD99, CD117, chromogranin, cytokeratin, desmin, glial fibrillary acidic protein (GFAP), gross cystic disease fluid protein (GCDFP-15), HMB-45 antigen, protein melan-A (melanoma antigen recognized by T lymphocytes; MART-1), myo-D1, muscle-specific actin (MSA), neurofilament, neuron-specific enolase (NSE), placental alkaline phosphatase, synaptophysin, thyroglobulin, thyroid transcription factor-1, the dimeric form of the pyruvate kinase isoenzyme type M2 (tumor M2-PK), an abnormal ras protein, or an abnormal p53 protein. In certain embodiments, the tumor-associated antigen is CD19, CD22, CD27, CD30, CD70, GD2 (ganglioside G2), EGFRvIII (epidermal growth factor variant III), sperm protein 17 (Sp17), mesothelin, PAP (prostatic acid phosphatase), prostein, TARP (T cell receptor gamma alternate reading frame protein), Trp-p8, orSTEAP1 (six-transmembrane epithelial antigen of the prostate 1).

In certain embodiments, the TAA or TSA is a cancer/testis (CT) antigen, e.g., BAGE, CAGE, CTAGE, FATE, GAGE, HCA661, HOM-TES-85, MAGEA, MAGEB, MAGEC, NA88, NY-ESO-1, NY-SAR-35, OY-TES-1, SPANXB1, SPA17, SSX, SYCP1, or TPTE.

In certain other embodiments, the TAA or TSA is a carbohydrate or ganglioside, e.g., fuc-GM1, GM2 (oncofetal antigen-immunogenic-1; OFA-I-1); GD2 (OFA-I-2), GM3, GD3, and the like.

In certain other embodiments, the TAA or TSA is alpha-actinin-4, Bage-1, BCR-ABL, Bcr-Abl fusion protein, beta-catenin, CA 125, CA 15-3 (CA 27.29\BCAA), CA 195, CA 242, CA-50, CAM43, Casp-8, cdc27, cdk4, cdkn2a, CEA, coa-1, dek-can fusion protein, EBNA, EF2, Epstein Barr virus antigens, ETV6-AML1 fusion protein, HLA-A2, HLA-A1 1, hsp70-2, KIAAO205, Mart2, Mum-1, 2, and 3, neo-PAP, myosin class I, OS-9, pml-RARα fusion protein, PTPRK, K-ras, N-ras, triosephosphate isomerase, Gage 3,4,5,6,7, GnTV, Herv-K-mel, Lage-1, NA-88, NY-Eso-1/Lage-2, SP17, SSX-2, TRP2-Int2, gp100 (Pmel 17), tyrosinase, TRP-1, TRP-2, MAGE-1, MAGE-3, RAGE, GAGE-1, GAGE-2, p15(58), RAGE, SCP-1, Hom/Mel-40, PRAME, p53, H-Ras, HER-2/neu, E2A-PRL, H4-RET, IGH-IGK, MYL-RAR, human papillomavirus (HPV) antigens E6 and E7, TSP-180, MAGE-4, MAGE-5, MAGE-6, p185erbB2, p180erbB-3, c-met, nm-23H1, PSA, TAG-72-4, CA 19-9, CA 72-4, CAM 17.1, NuMa, K-ras, 13-Catenin, Mum-1, p16, TAGE, PSMA (prostate-specific membrane antigen), B cell maturation antigen (BCMA), CT7, telomerase, 43-9F, 5T4, 791Tgp72, 13HCG, BCA225, BTAA, CD68\KP1, CO-029, FGF-5, G250, Ga733 (EpCAM), HTgp-175, M344, MA-50, MG7-Ag, MOV18, NB\70K, NY-CO-1, RCAS1, SDCCAG16, TA-90, TAAL6, TAG72, TLP, or TPS.

In another specific embodiment, said first antigen is integrin αvβ3 (CD61), galactin, K-Ras (V-Ki-ras2 Kirsten rat sarcoma viral oncogene) or Ral-B.

Other tumor-associated and tumor-specific antigens are known to those in the art and may be targeted by the modified T lymphocytes provided herein.

In certain embodiments in which the second antigen bound by the artificial polypeptide's antigen binding domain is not a TSA or TAA, the antigen can be, e.g., a growth factor, cytokine or interleukin, e.g., a growth factor, cytokine, or interleukin associated with angiogenesis or vasculogenesis. Such growth factors, cytokines, or interleukins can include, e.g., vascular endothelial growth factor (VEGF), basic fibroblast growth factor (bFGF), platelet-derived growth factor (PDGF), hepatocyte growth factor (HGF), insulin-like growth factor (IGF), or interleukin-8 (IL-8).

Tumors can also create a hypoxic environment local to the tumor. As such, in other more specific embodiments, signal transduction by said artificial polypeptide (e.g., chimeric receptor) is induced by activation of a hypoxia-associated factor, e.g., HIF-1α, HIF-1β, HIF-2α, HIF-2β, HIF-3α, or HIF-3β, or is otherwise induced by hypoxic response element activation.

Tumors can also cause localized damage to normal tissue, causing the release of molecules known as damage associated molecular pattern molecules (DAMPs; also known as alarmins). In certain embodiments, the second antigen is a DAMP, e.g., a heat shock protein, chromatin-associated protein high mobility group box 1 (HMGB 1), S100A8 (MRP8, calgranulin A), S100A9 (MRP14, calgranulin B), serum amyloid A (SAA), deoxyribonucleic acid, adenosine triphosphate, uric acid, or heparin sulfate.

### 4.1.2. Second Configuration, Basic Structure

In one aspect, provided herein is a modified T lymphocyte comprising a first polypeptide comprising a first extracellular antigen binding domain that binds a first antigen, and a first intracellular signaling domain, wherein said first polypeptide does not comprise a co-stimulatory domain; and a second polypeptide comprising a second extracellular antigen binding domain binding a second antigen, or a receptor that binds said second antigen; and a second intracellular signaling domain; wherein said modified lymphocyte becomes maximally cytotoxic only when said first signaling domain and said second signaling domain are both activated by said first antigen and said second antigen, respectively. Typically, the two polypeptides are both chimeric receptors. Binding of said first antigen to said first antigen binding domain without binding of said second antigen to said second binding domain, or binding of said second antigen to said second antigen binding domain without binding of first second antigen to said first binding domain either induces anergy of said modified T lymphocyte, or renders the modified T lymphocyte nonresponsive to the binding of the first antigen to the first antigen binding domain alone.

The antigen binding portions of the first polypeptide and the second polypeptide can, independently, be any polypeptide domain, motif or sequence that binds to an antigen. said first antigen binding domain and said second antigen binding domain are independently an antigen binding portion of a receptor or an antigen-binding portion of an antibody. For example, the first and second antigen-binding domains can be receptors or an antigen binding portion thereof, e.g., a receptor for a ligand produced by a tumor cell, an antibody, antibody chain, or an antigen binding portion thereof, an Fc domain, a glycophosphatidylinositol anchor domain, or the like. In certain embodiments, therefore, either or both of said first antigen binding domain or said second antigen binding domain are scFv antibody fragments. In certain other embodiments, the first and second antigen-binding domains can be another form of peptide-based macromolecular antigen binding agent, e.g., phage display proteins. In various embodiments, antigen binding by the antigen binding domains can be restricted to antigen presentation in association with major histocompatibility complexes (MHC), or can be MHC-unrestricted.

In addition to the extracellular and intracellular portions, said first polypeptide and/or said second polypeptide preferably additionally comprise a transmembrane domain. The transmembrane domain can be obtained or derived from the transmembrane domain of any transmembrane protein, and can include all or a portion of such transmembrane domain. In specific embodiments, the transmembrane domain can be obtained or derived from, e.g., CD16, a cytokine receptor, and interleukin receptor, or a growth factor receptor, or the like.

The first polypeptide or said second polypeptide may also comprise a T cell survival motif. The T cell survival motif can be any polypeptide sequence or motif that facilitates the survival of the T lymphocyte after stimulation by an antigen. In certain embodiments, the T cell survival motif is, or is derived from, CD3, CD28, an intracellular signaling domain of IL-7 receptor (IL-7R), an intracellular signaling domain of IL-12 receptor, an intracellular signaling domain of IL-15 receptor, an intracellular signaling domain of IL-21 receptor, or an intracellular signaling domain of transforming growth factor ß (TGFß) receptor.

The first intracellular signaling domain of the first polypeptide can be any polypeptide that is capable of transmitting an antigen binding signal from the first antigen binding domain of the first polypeptide, e.g., in a manner similar to the CD3ζ (CD3 zeta) chains of the native T lymphocyte receptor. In certain embodiments, the first intracellular signaling domain comprises a polypeptide sequence comprising an immunoreceptor tyrosine-based activation motif (ITAM). Preferably, the polypeptide sequence is a CD3ζ signaling domain or a signal-transducing variant thereof.

The second polypeptide comprises one or more co-stimulatory domains, enabling the second polypeptide to provide co-stimulation when the second antigen binds to the second antigen-binding domain of the second polypeptide. Any co-stimulatory motif, or functional portion thereof, may be used. In certain specific embodiments, the one or more co-stimulatory domains comprises one or more of a co-stimulatory CD27 polypeptide sequence, a co-stimulatory CD28 polypeptide sequence, a co-stimulatory OX40 (CD134) polypeptide sequence, a co-stimulatory 4-1BB (CD137) polypeptide sequence, or a co-stimulatory inducible T-cell costimulatory (ICOS) polypeptide sequence.

The first antigen may be any antigen of interest, e.g., an antigen that is expressed on the surface of a cell. In preferred embodiments, said first antigen is an antigen on a tumor cell, e.g., a TSA or TAA, e.g., any of the TSAs or TAAs disclosed in Section 5.1, above. The tumor cell can be a cell, e.g., of a solid tumor or a blood cancer.

The antigen can be any antigen that is expressed on a cell of any tumor or cancer type, e.g., cells of a lymphoma, a lung cancer, a breast cancer, a prostate cancer, an adrenocortical carcinoma, a thyroid carcinoma, a nasopharyngeal carcinoma, a melanoma, e.g., a malignant melanoma, a skin carcinoma, a colorectal carcinoma, a desmoid tumor, a desmoplastic small round cell tumor, an endocrine tumor, an Ewing sarcoma, a peripheral primitive neuroectodermal tumor, a solid germ cell tumor, a hepatoblastoma, a neuroblastoma, a non-rhabdomyosarcoma soft tissue sarcoma, an osteosarcoma, a retinoblastoma, a rhabdomyosarcoma, a Wilms tumor, a glioblastoma, a myxoma, a fibroma, a lipoma, or the like. In more specific embodiments, said lymphoma can be chronic lymphocytic leukemia (small lymphocytic lymphoma), B-cell prolymphocytic leukemia, lymphoplasmacytic lymphoma, Waldenstrom macroglobulinemia, splenic marginal zone lymphoma, plasma cell myeloma, plasmacytoma, extranodal marginal zone B cell lymphoma, MALT lymphoma, nodal marginal zone B cell lymphoma, follicular lymphoma, mantle cell lymphoma, diffuse large B cell lymphoma, mediastinal (thymic) large B cell lymphoma, intravascular large B cell lymphoma, primary effusion lymphoma, Burkitt's lymphoma, T lymphocyte prolymphocytic leukemia, T lymphocyte large granular lymphocytic leukemia, aggressive NK cell leukemia, adult T lymphocyte leukemia/lymphoma, extranodal NK/T lymphocyte lymphoma, nasal type, enteropathy-type T lymphocyte lymphoma, hepatosplenic T lymphocyte lymphoma, blastic NK cell lymphoma, mycosis fungoides, Sezary syndrome, primary cutaneous anaplastic large cell lymphoma, lymphomatoid papulosis, angioimmunoblastic T lymphocyte lymphoma, peripheral T lymphocyte lymphoma (unspecified), anaplastic large cell lymphoma, Hodgkin lymphoma, or a non-Hodgkin lymphoma.

The second antigen can be any antigen different from the first antigen, but is preferably related to the first antigen. For example, both the first and second antigens can be tumor-associated antigens or tumor-specific antigens that are present on the same tumor cell type. Preferably, the first antigen is a tumor-associated antigen or a tumor-specific antigens, and said second antigen is not a tumor-associated antigen or a tumor-specific antigen. In such an embodiment, the second antigen, in certain embodiments, is related to an aspect of the tumor, e.g., the tumor environment. For example, a tumor can induce an inflammatory state in tissue surrounding the tumor, and can release angiogenic growth factors, interleukins, and/or cytokines that promote angiogenesis into and at the periphery of the tumor. Thus, in specific embodiments, the second antigen is a growth factor, cytokine or interleukin, e.g., a growth factor, cytokine, or interleukin associated with angiogenesis or vasculogenesis. Such growth factors, cytokines, or interleukins can include, e.g., vascular endothelial growth factor (VEGF), basic fibroblast growth factor (bFGF), platelet-derived growth factor (PDGF), hepatocyte growth factor (HGF), insulin-like growth factor (IGF), or interleukin-8 (IL-8).

Tumors can also create a hypoxic environment local to the tumor. As such, in other more specific embodiments, signal transduction by said second chimeric receptor is induced by activation of a hypoxia-associated factor, e.g., HIF-1α, HIF-1β, HIF-2α, HIF-2β, HIF-3α, or HIF-3β, or is otherwise induced by hypoxic response element activation.

Tumors can also cause localized damage to normal tissue, causing the release of molecules known as damage associated molecular pattern molecules (DAMPs; also known as alarmins). In certain embodiments, the second antigen is a DAMP, e.g., a heat shock protein, chromatin-associated protein high mobility group box 1 (HMGB1), S100A8 (MRP8, calgranulin A), S100A9 (MRP14, calgranulin B), serum amyloid A (SAA), deoxyribonucleic acid, adenosine triphosphate, uric acid, or heparin sulfate.

It is possible to direct a chimeric receptor, e.g., the second chimeric receptor polypeptide, to an antigen that is not native to the antigen or to the surrounding tissue. For example, tumor cells, or cells of surrounding normal tissue, may be contacted with an antibody that binds to at least one antigen on the cells. In this case, any antigens on the antibody itself can bind to the second antigen-binding portion of the second chimeric receptor polypeptide. In certain embodiments, the first antigen, that binds to the first antigen-binding portion of the first polypeptide, is an antigen on an antibody that binds to an antigen presented by a tumor cell. In certain embodiments, the second antigen, that binds to the second antigen-binding portion of the second polypeptide, is an antigen on an antibody that binds to an antigen presented by a tumor cell. In certain embodiments, the first antigen is an antigen on a first antibody, and the second antigen is an antigen on a second antibody. In such an embodiment, the first antibody can be an antibody that binds to, e.g., a tumor-associated antigen or a tumor-specific antigen, and the second antibody is an antibody to a cytokine, interleukin, growth factor, DAMP, or other non-TAA, non-TSA protein associated with the tumor.

### 4.1.3. Specific Embodiments

Thus, in one configuration, provided herein are modified T lymphocytes comprising: a) a first polypeptide comprising a first extracellular antigen binding domain that binds a first antigen, and an intracellular CD3ζ signaling domain; and b) a second polypeptide comprising an extracellular antigen binding domain that binds an angiogenic or vasculogenic factor, and a second intracellular signaling domain, wherein said first polypeptide does not comprise a co-stimulatory domain; wherein said modified lymphocyte becomes maximally cytotoxic only when said first signaling domain and said second signaling domain are both activated by said first antigen and said second antigen, respectively.

In another configuration, provided herein are modified T lymphocytes comprising: a) a first polypeptide comprising an scFv or antigen-binding portion thereof that binds a first antigen, and an intracellular CD3ζ signaling domain, wherein said first polypeptide does not comprise a co-stimulatory domain; and b) a second polypeptide comprising an extracellular antigen binding domain that binds to an angiogenic or vasculogenic factor, and a second intracellular signaling domain; wherein said modified lymphocyte becomes maximally cytotoxic only when said first signaling domain and said second signaling domain are both activated by said first antigen and said second antigen, respectively.

In a more specific configuration, provided herein are modified T lymphocytes comprising: a) a first polypeptide comprising an extracellular antigen binding domain that binds a first antigen, wherein said first antigen is a tumor-associated antigen (TAA) or tumor-specific antigen (TSA), and an intracellular CD3ζ signaling domain, wherein said first polypeptide does not comprise a co-stimulatory domain; and b) a second polypeptide comprising a second extracellular antigen binding domain that binds to a second antigen, wherein said second antigen is VEGF, bFGF, PDGF, HGF, IGF, or IL-8, and a second intracellular signaling domain; wherein said modified lymphocyte becomes maximally cytotoxic only when said first signaling domain and said second signaling domain are both activated by said first antigen and said second antigen, respectively.

In another more specific configuration, provided herein are modified T lymphocytes comprising: a) a first polypeptide comprising a first extracellular antigen binding domain that binds a first antigen, wherein said first antigen is a TAA or TSA, and a first intracellular CD3ζ signaling domain, wherein said first polypeptide does not comprise a co-stimulatory domain; and b) a second polypeptide comprising a second extracellular antigen binding domain that binds a second antigen, wherein said second antigen is VEGF, bFGF, PDGF, HGF, IGF, or IL-8, and a second intracellular signaling domain comprising a co-stimulatory signaling domain from one or more of CD27, CD28, OX40, ICOS, and 4-1BB ; wherein said modified lymphocyte becomes maximally cytotoxic only when said first signaling domain and said second signaling domain are both activated by said first antigen and said second antigen, respectively.

In another more specific configuration, provided herein are modified T lymphocytes comprising: a) a first polypeptide comprising an extracellular antigen binding domain that binds a first antigen, wherein said first antigen is Her2, PSCA, PSMA, BCMA, ERK5, AFP, CEA, CA-125, CA19-9, calretinin, MUC-1, EMA, ETA, tyrosinase, MAGE, CD34, CD45, CD99, CD117, chromogranin, cytokeratin, desmin, GFAP, GCDFP-15, HMB-45 antigen, MART-1, myo-D1, MSA, neurofilament, NSE, placental alkaline phosphatase, synaptophysin, thyroglobulin, thyroid transcription factor-1, tumor M2-PK, CD19, CD22, CD27, CD30, CD70, GD2 (ganglioside G2), EGFRvIII (epidermal growth factor variant III), sperm protein 17 (Sp17), mesothelin, PAP (prostatic acid phosphatase), prostein, TARP (T cell receptor gamma alternate reading frame protein), Trp-p8, STEAP1 (six-transmembrane epithelial antigen of the prostate 1), an abnormal ras protein, or an abnormal p53 protein; and an intracellular CD3ζ signaling domain, wherein said first polypeptide does not comprise a co-stimulatory domain; and b) a second polypeptide comprising an extracellular antigen binding domain that binds to a second antigen, wherein said second antigen is VEGF, bFGF, PDGF, HGF, IGF, or IL-8, and a second intracellular signaling domain comprising co-stimulatory signaling domains from one or more of CD27, CD28, OX40, ICOS, and 4-1BB ; wherein said modified lymphocyte becomes maximally cytotoxic only when said first signaling domain and said second signaling domain are both activated by said first antigen and said second antigen, respectively.

In another more specific configuration, provided herein are modified T lymphocytes comprising: a) a first polypeptide comprising an extracellular antigen binding domain that binds a first antigen, wherein said first antigen is Her2, PSCA, PSMA, BCMA, ERK5, AFP, CEA, CA-125, CA19-9, calretinin, MUC-1, EMA, ETA, tyrosinase, MAGE, CD34, CD45, CD99, CD117, chromogranin, cytokeratin, desmin, GFAP, GCDFP-15, HMB-45 antigen, MART-1, myo-D1, MSA, neurofilament, NSE, placental alkaline phosphatase, synaptophysin, thyroglobulin, thyroid transcription factor-1, tumor M2-PK, CD19, CD22, CD27, CD30, CD70, GD2 (ganglioside G2), EGFRvIII (epidermal growth factor variant III), sperm protein 17 (Sp17), mesothelin, PAP (prostatic acid phosphatase), prostein, TARP (T cell receptor gamma alternate reading frame protein), Trp-p8, STEAP1 (six-transmembrane epithelial antigen of the prostate 1), an abnormal ras protein, or an abnormal p53 protein, and an intracellular CD3ζ signaling domain, wherein said first polypeptide does not comprise a co-stimulatory domain; and b) a second polypeptide comprising a second extracellular antigen binding domain that binds to a second antigen, wherein said second antigen is VEGF, bFGF, PDGF, HGF, IGF, or IL-8, and an intracellular signaling domain comprising co-stimulatory signaling domains from each of CD27, CD28, OX40, ICOS, and 4-1BB; wherein said modified lymphocyte becomes maximally cytotoxic only when said first signaling domain and said second signaling domain are both activated by said first antigen and said second antigen, respectively.

In another more specific configuration, provided herein are modified T lymphocytes comprising: a) a first polypeptide comprising an scFv or antigen binding portion thereof that binds a first antigen, wherein said first antigen is Her2, PSCA, PSMA, BCMA, ERK5, AFP, CEA, CA-125, CA19-9, calretinin, MUC-1, EMA, ETA, tyrosinase, MAGE, CD34, CD45, CD99, CD117, chromogranin, cytokeratin, desmin, GFAP, GCDFP-15, HMB-45 antigen, MART-1, myo-D1, MSA, neurofilament, NSE, placental alkaline phosphatase, synaptophysin, thyroglobulin, thyroid transcription factor-1, tumor M2-PK, CD19, CD22, CD27, CD30, CD70, GD2 (ganglioside G2), EGFRvIII (epidermal growth factor variant III), sperm protein 17 (Sp17), mesothelin, PAP (prostatic acid phosphatase), prostein, TARP (T cell receptor gamma alternate reading frame protein), Trp-p8, STEAP1 (six-transmembrane epithelial antigen of the prostate 1), an abnormal ras protein, or an abnormal p53 protein, and an intracellular CD3ζ signaling domain, wherein said first polypeptide does not comprise a co-stimulatory domain; and b) a second polypeptide comprising an extracellular antigen binding domain that binds to a second antigen, wherein said second antigen is VEGF, bFGF, PDGF, HGF, IGF, or IL-8, and a second intracellular signaling domain comprising co-stimulatory signaling domains from each of CD27, CD28, OX40, ICOS, and 4-1BB; wherein said modified lymphocyte becomes maximally cytotoxic only when said first signaling domain and said second signaling domain are both activated by said first antigen and said second antigen, respectively.

In any of the above specific configurations, either or both of said first polypeptide or said second polypeptide comprises a T cell survival motif, e.g., a T cell survival motif from CD28, IL-7R, IL-12R, IL-15R, IL-21R, TGFßR.

### 4.1.4. Third Configuration, Basic Structure

The two polypeptides (e.g., chimeric receptors) comprised within the modified T lymphocytes can be constructed so that binding of the first antigen, e.g., a TAA or TSA, to the first antigen binding domain of the first polypeptide produces not a primary antigen-binding signal, but a co-stimulatory signal, and binding of a second antigen produces the primary antigen-binding signal. Such a configuration would enjoy the same advantages as the first configuration, above, in that two antigen binding events must take place in order to fully activate the T lymphocyte.

Thus, provided herein is a modified T lymphocyte comprising: a) a first polypeptide comprising a first extracellular antigen binding domain that binds a first antigen, and a first intracellular signaling domain; and b) a second polypeptide comprising a second extracellular antigen binding domain binding a second antigen; and a second intracellular signaling domain, wherein said second polypeptide does not comprise a co-stimulatory domain; wherein said modified lymphocyte becomes maximally cytotoxic only when said first signaling domain and said second signaling domain are both activated by said first antigen and said second antigen, respectively. As above, the first antigen and the second antigen are different antigens. In a specific embodiment, binding of said first antigen to said first antigen binding domain without binding of said second antigen to said second binding domain, or binding of said second antigen to said second antigen binding domain without binding of first second antigen to said first binding domain induces anergy of said modified T lymphocyte.

In certain embodiments, said first antigen binding domain and said second antigen binding domain are independently any antigen binding domain, e.g., any of the antigen binding domains disclosed in Section 5.2, above, e.g., an antigen-binding portion of a receptor or an antigen-binding portion of an antibody. In a more specific embodiment, either or both of said first antigen binding domain or said second antigen binding domain are scFv antibody fragments.

The first polypeptide comprises one or more co-stimulatory domains, enabling the first polypeptide to provide co-stimulation when the first antigen binds to the first antigen-binding domain of the first polypeptide. Any co-stimulatory motif, or functional portion thereof, may be used. In certain specific embodiments, the one or more co-stimulatory domains comprises one or more of a co-stimulatory CD27 polypeptide sequence, a co-stimulatory CD28 polypeptide sequence, a co-stimulatory OX40 (CD134) polypeptide sequence, a co-stimulatory 4-1BB (CD137) polypeptide sequence, or a co-stimulatory inducible T-cell co-stimulatory (ICOS) polypeptide sequence.

The second intracellular signaling domain of the second polypeptide can be any polypeptide that is capable of transmitting an antigen binding signal from the second antigen binding domain of the second polypeptide, e.g., in a manner similar to the CD3ζ (CD3 zeta) chains of the native T lymphocyte receptor. In certain embodiments, the second intracellular signaling domain comprises a polypeptide sequence comprising an immunoreceptor tyrosine-based activation motif (ITAM). Preferably, the polypeptide sequence is a CD3ζ signaling domain or a signal-transducing variant thereof.

In certain embodiments, either said first polypeptide or said second polypeptide additionally comprises a transmembrane domain. In certain embodiments, the first polypeptide comprises one or more co-stimulatory domains, enabling the first polypeptide to provide co-stimulation when the first antigen binds to the first antigen-binding domain of the second polypeptide. Any co-stimulatory domain, or functional portion thereof, may be used, e.g., co-stimulatory signaling domains from each of CD27, CD28, OX40, ICOS, and 4-1BB. In certain other embodiments, said first polypeptide or said second polypeptide comprises a T cell survival motif.

In this configuration, the first antigen may be any antigen of interest, e.g., an antigen that is expressed on the surface of a cell. In preferred embodiments, said first antigen is an antigen on a tumor cell. The tumor cell can be a cell, e.g., of a solid tumor or a blood cancer, e.g., any of the cancer or tumor types disclosed in Section 5.2, above. In certain specific embodiments, said antigen is a TAA or TSA, e.g., any of the TAAs or TSAs disclosed in Section 5.1, above.

The second antigen can be any antigen different from the first antigen, but is preferably related to the first antigen. For example, both the first and second antigens can be tumor-associated antigens or tumor-specific antigens that are present on the same tumor cell type. Preferably, the first antigen is a TAA or TSA, and said second antigen is not a TAA or TSA. In such an embodiment, the second antigen, in certain embodiments, can be related to an aspect of the tumor, e.g., the tumor environment. For example, a tumor can induce an inflammatory state in tissue surrounding the tumor, and can release angiogenic growth factors, interleukins, and/or cytokines that promote angiogenesis into and at the periphery of the tumor. Thus, in specific embodiments, the second antigen is a growth factor, cytokine or interleukin, e.g., a growth factor, cytokine, or interleukin associated with angiogenesis or vasculogenesis, e.g., VEGF, bFGF, PDGF, HGF, IGF, or IL-8. In other more specific embodiments, signal transduction by said second chimeric receptor is induced by activation of a hypoxia-associated factor, e.g., HIF-1α, HIF-1β, HIF-2α, HIF-2β, HIF-3α, or HIF-3β. In certain other embodiments, the second antigen is a DAMP, e.g., a heat shock protein, HMGB1, S100A8 (MRP8, calgranulin A), S100A9 (MRP14, calgranulin B), SAA, deoxyribonucleic acid, adenosine triphosphate, uric acid, or heparin sulfate.

### 4.1.5. Specific Embodiments

Thus, in one configuration, provided herein are modified T lymphocytes comprising: a) a first polypeptide comprising a first extracellular antigen binding domain that binds a first antigen, and a first intracellular signaling domain; and b) a second polypeptide comprising a second extracellular antigen binding domain that binds an angiogenic or vasculogenic factor, and an intracellular CD3ζ signaling domain, wherein said second polypeptide does not comprise a co-stimulatory domain; wherein said modified lymphocyte becomes maximally cytotoxic only when said first signaling domain and said second signaling domain are both activated by said first antigen and said second antigen, respectively.

In another configuration, provided herein are modified T lymphocytes comprising: a) a first polypeptide comprising an scFv or antigen-binding portion thereof, which binds a first antigen, and a first intracellular signaling domain; and b) a second polypeptide comprising a second extracellular antigen binding domain binding an angiogenic or vasculogenic factor, and an intracellular CD3ζ signaling domain, wherein said second polypeptide does not comprise a co-stimulatory domain; wherein said modified lymphocyte becomes maximally cytotoxic only when said first signaling domain and said second signaling domain are both activated by said first antigen and said second antigen, respectively.

In a more specific configuration, provided herein are modified T lymphocytes comprising: a) a first polypeptide comprising a first extracellular antigen binding domain that binds a first antigen, wherein said first antigen is a tumor-associated antigen (TAA) or tumor-specific antigen (TSA), and a first intracellular signaling domain; and b) a second polypeptide comprising a second extracellular antigen binding domain that binds a second antigen, wherein said second antigen is VEGF, bFGF, PDGF, HGF, IGF, or IL-8, and an intracellular CD3ζ signaling domain, wherein said second polypeptide does not comprise a co-stimulatory domain; wherein said modified lymphocyte becomes maximally cytotoxic only when said first signaling domain and said second signaling domain are both activated by said first antigen and said second antigen, respectively.

In another more specific configuration, provided herein are modified T lymphocytes comprising: a) a first polypeptide comprising a first extracellular antigen binding domain that binds a first antigen, wherein said first antigen is a TAA or TSA, and a first intracellular signaling domain comprising co-stimulatory signaling domains from one or more of CD27, CD28, OX40, ICOS, and 4-1BB; and b) a second polypeptide comprising a second extracellular antigen binding domain that binds a second antigen, wherein said second antigen is VEGF, bFGF, PDGF, HGF, IGF, or IL-8, and an intracellular CD3ζ signaling domain, wherein said second polypeptide does not comprise a co-stimulatory domain; wherein said modified lymphocyte becomes maximally cytotoxic only when said first signaling domain and said second signaling domain are both activated by said first antigen and said second antigen, respectively.

In another more specific configuration, provided herein are modified T lymphocytes comprising: a) a first polypeptide comprising an extracellular antigen binding domain that binds a first antigen, wherein said first antigen is Her2, PSCA, PSMA, BCMA, ERK5, AFP, CEA, CA-125, CA19-9, calretinin, MUC-1, EMA, ETA, tyrosinase, MAGE, CD34, CD45, CD99, CD117, chromogranin, cytokeratin, desmin, GFAP, GCDFP-15, HMB-45 antigen, MART-1, myo-D1, MSA, neurofilament, NSE, placental alkaline phosphatase, synaptophysin, thyroglobulin, thyroid transcription factor-1, tumor M2-PK, CD19, CD22, CD27, CD30, CD70, GD2 (ganglioside G2), EGFRvIII (epidermal growth factor variant III), sperm protein 17 (Sp17), mesothelin, PAP (prostatic acid phosphatase), prostein, TARP (T cell receptor gamma alternate reading frame protein), Trp-p8, STEAP1 (six-transmembrane epithelial antigen of the prostate 1), an abnormal ras protein, or an abnormal p53 protein; and an intracellular signaling domain comprising co-stimulatory signaling domains from one or more of CD27, CD28, OX40, ICOS, and 4-1BB; and b) a second polypeptide comprising an extracellular antigen binding domain that binds a second antigen, wherein said second antigen is VEGF, bFGF, PDGF, HGF, IGF, or IL-8, and an intracellular CD3ζ signaling domain, wherein said second polypeptide does not comprise a co-stimulatory domain; wherein said modified lymphocyte becomes maximally cytotoxic only when said first signaling domain and said second signaling domain are both activated by said first antigen and said second antigen, respectively.

In another more specific configuration, provided herein are modified T lymphocytes comprising: a) a first polypeptide comprising a first extracellular antigen binding domain that binds a first antigen, wherein said first antigen is Her2, PSCA, PSMA, BCMA, ERK5, AFP, CEA, CA-125, CA19-9, calretinin, MUC-1, EMA, ETA, tyrosinase, MAGE, CD34, CD45, CD99, CD117, chromogranin, cytokeratin, desmin, GFAP, GCDFP-15, HMB-45 antigen, MART-1, myo-D1, MSA, neurofilament, NSE, placental alkaline phosphatase, synaptophysin, thyroglobulin, thyroid transcription factor-1, tumor M2-PK, CD19, CD22, CD27, CD30, CD70, GD2 (ganglioside G2), EGFRvIII (epidermal growth factor variant III), sperm protein 17 (Sp17), mesothelin, PAP (prostatic acid phosphatase), prostein, TARP (T cell receptor gamma alternate reading frame protein), Trp-p8, STEAP1 (six-transmembrane epithelial antigen of the prostate 1), an abnormal ras protein, or an abnormal p53 protein, and an intracellular signaling domain comprising co-stimulatory signaling domains from each of CD27, CD28, OX40, ICOS, and 4-1BB; and b) a second polypeptide comprising a second extracellular antigen binding domain that binds a second antigen, wherein said second antigen is VEGF, bFGF, PDGF, HGF, IGF, or IL-8, and an intracellular CD3ζ signaling domain, wherein said second polypeptide does not comprise a co-stimulatory domain; wherein said modified lymphocyte becomes maximally cytotoxic only when said first signaling domain and said second signaling domain are both activated by said first antigen and said second antigen, respectively.

In another more specific configuration, provided herein are modified T lymphocytes comprising a first polypeptide comprising: a) a first extracellular antigen binding domain that binds a first antigen, wherein said first extracellular antigen binding domain is an csFv or antigen binding portion thereof, and wherein said first antigen is Her2, PSCA, PSMA, BCMA, ERK5, AFP, CEA, CA-125, CA19-9, calretinin, MUC-1, EMA, ETA, tyrosinase, MAGE, CD34, CD45, CD99, CD117, chromogranin, cytokeratin, desmin, GFAP, GCDFP-15, HMB-45 antigen, MART-1, myo-D1, MSA, neurofilament, NSE, placental alkaline phosphatase, synaptophysin, thyroglobulin, thyroid transcription factor-1, tumor M2-PK, CD19, CD22, CD27, CD30, CD70, GD2 (ganglioside G2), EGFRvIII (epidermal growth factor variant III), sperm protein 17 (Sp17), mesothelin, PAP (prostatic acid phosphatase), prostein, TARP (T cell receptor gamma alternate reading frame protein), Trp-p8, STEAP1 (six-transmembrane epithelial antigen of the prostate 1), an abnormal ras protein, or an abnormal p53 protein; and an intracellular signaling domain comprising co-stimulatory signaling domains from each of CD27, CD28, OX40, ICOS, and 4-1BB; and b) a second polypeptide comprising an extracellular antigen binding domain that binds a second antigen, wherein said second antigen is VEGF, bFGF, PDGF, HGF, IGF, or IL-8, and an intracellular CD3ζ signaling domain, wherein said first polypeptide does not comprise a co-stimulatory domain; wherein said modified lymphocyte becomes maximally cytotoxic only when said first signaling domain and said second signaling domain are both activated by said first antigen and said second antigen, respectively.

In any of the above specific configurations, either or both of said first polypeptide or said second polypeptide comprises a T cell survival motif, e.g., a T cell survival motif from CD28, IL-7R, IL-12R, IL-15R, IL-21R, TGFßR.

### 4.1.6. Fourth Configuration, Basic Structure

In a fourth configuration, the two chimeric receptors contained within the modified T lymphocytes can be constructed so that a first chimeric receptor directed to a first antigen comprises both primary, antigen binding signaling domains and co-stimulatory domains, but no polypeptide sequence comprising a T cell survival motif, while a second chimeric receptor, directed to a second antigen, comprises a polypeptide sequence comprising a T cell survival motif. In this configuration, T lymphocytes are directed to cells expressing a desired antigen, and, upon antigen binding, antigen binding and co-stimulatory signals are generated; however, in the absence of the binding of the second chimeric receptor to a second antigen, T lymphocytes are not directed to survive. As such, off-tumor effects are, again, eliminated or mitigated.

Thus, provided herein is a modified T lymphocyte comprising: a) a first polypeptide comprising a first extracellular antigen binding domain that binds a first antigen, a signaling domain, and one or more co-stimulatory motifs; and b) a second polypeptide comprising a second extracellular antigen binding domain that binds a second antigen, and a T cell survival motif, wherein said second polypeptide does not comprise a co-stimulatory motif; wherein said modified lymphocyte survives only when said signaling domain and said T cell survival motif are both activated by said first antigen and said second antigen, respectively. In certain embodiments, said T cell survival motif is an IL-7 receptor intracellular T cell survival motif is a T cell survival motif from CD28, IL-7R, IL-12R, IL-15R, IL-21R, or TGFβR. In certain embodiments, either or both of said first polypeptide or said second polypeptide comprise a transmembrane domain. In a more specific embodiment, said second polypeptide comprises a domain of CD27, CD28, IL-7R, IL-12R, IL-15R, IL-21R, or TGFßR that comprises a T cell survival motif.

As above, structurally the first antigen binding domain and said second antigen binding domain are independently any antigen binding domain, e.g., any of the antigen binding domains disclosed in Section 5.2, above, e.g., an antigen-binding portion of a receptor or an antigen-binding portion of an antibody. In a more specific embodiment, either or both of said first antigen binding domain or said second antigen binding domain are scFv antibody fragments. The first antigen may be any antigen of interest, e.g., an antigen that is expressed on the surface of a cell. In preferred embodiments, said first antigen is an antigen on a tumor cell. The tumor cell can be a cell, e.g., of a solid tumor or a blood cancer, e.g., any of the cancer or tumor types disclosed in Section 5.2, above. In certain specific embodiments, said antigen is a TAA or TSA, e.g., any of the TAAs or TSAs disclosed in Section 5.1, above. The second antigen, which is different from the first antigen, can be an angiogenic or vasculogenic factor, e.g., any of the angiogenic or vasculogenic factors disclosed in Section 5.1, above; or any DAMP, e.g., the DAMPs disclosed in Section 5.1, above. In other specific embodiments, signal transduction by said second chimeric receptor is induced by activation of a hypoxia-associated factor, e.g., HIF- 1α, HIF-1β, HIF-2α, HIF-2β, HIF-3α, or HIF-3β.

The first intracellular signaling domain of the first polypeptide can be any polypeptide that is capable of transmitting an antigen binding signal from the first antigen binding domain of the first polypeptide, e.g., in a manner similar to the CD3ζ (CD3 zeta) chains of the native T lymphocyte receptor. In certain embodiments, the second intracellular signaling domain comprises a polypeptide sequence comprising an immunoreceptor tyrosine-based activation motif (ITAM). Preferably, the polypeptide sequence is a CD3ζ signaling domain or a signal-transducing variant thereof. The first polypeptide additionally comprises one or more co-stimulatory domains, e.g., any co-stimulatory motif or functional portion thereof, e.g., a co-stimulatory CD27, CD28, OX40 (CD134), 4-1BB (CD137), or ICOS polypeptide sequence.

### 4.1.7. Specific Embodiments

In one configuration, provided herein are modified T lymphocytes comprising: a) a first polypeptide comprising a first extracellular antigen binding domain that binds a first antigen, an intracellular CD3ζ signaling domain, and one or more co-stimulatory motifs; and b) a second polypeptide comprising a second extracellular antigen binding domain that binds a second antigen, and a T cell survival motif, wherein said second polypeptide does not comprise a co-stimulatory motif; wherein said modified lymphocyte survives only when said signaling domain and said T cell survival motif are both activated by said first antigen and said second antigen, respectively.

In another configuration, provided herein are modified T lymphocytes comprising: a) a first polypeptide comprising a first extracellular antigen binding domain that binds a first antigen, an intracellular CD3ζ signaling domain, and a co-stimulatory polypeptide sequence from CD27, CD28, OX40 (CD134), 4-1BB (CD137), or ICOS; and b) a second polypeptide comprising a second extracellular antigen binding domain that binds a second antigen, and a T cell survival motif, wherein said second polypeptide does not comprise a co-stimulatory motif; wherein said modified lymphocyte survives only when said signaling domain and said T cell survival motif are both activated by said first antigen and said second antigen, respectively.

In another configuration, provided herein are modified T lymphocytes comprising: a) a first polypeptide comprising a first extracellular antigen binding domain that binds a first antigen, an intracellular CD3ζ signaling domain, and a co-stimulatory polypeptide sequence from CD27, CD28, OX40 (CD134), 4-1BB (CD137), or ICOS; and b) a second polypeptide comprising a second extracellular antigen binding domain that binds a second antigen, wherein said second antigen is an angiogenic or vasculogenic factor, or a DAMP, and a T cell survival motif, wherein said second polypeptide does not comprise a co-stimulatory motif; wherein said modified lymphocyte survives only when said signaling domain and said T cell survival motif are both activated by said first antigen and said second antigen, respectively. In other more specific embodiments, signal transduction by said second chimeric receptor is induced by activation of a hypoxia-associated factor, e.g., HIF-1α, HIF-1β, HIF-2α, HIF-2β, HIF-3α, or HIF-3β.

In another configuration, provided herein are modified T lymphocytes comprising: a) a first polypeptide comprising a first extracellular antigen binding domain that binds a first antigen, an intracellular CD3ζ signaling domain, and a co-stimulatory polypeptide sequence from CD27, CD28, OX40 (CD134), 4-1BB (CD137), or ICOS; and b) a second polypeptide comprising a second extracellular antigen binding domain that binds a second antigen, wherein said second antigen is VEGF, bFGF, PDGF, HGF, IGF, or IL-8, and a T cell survival motif, wherein said second polypeptide does not comprise a co-stimulatory motif; wherein said modified lymphocyte survives only when said signaling domain and said T cell survival motif are both activated by said first antigen and said second antigen, respectively.

In another configuration, provided herein are modified T lymphocytes comprising: a) a first polypeptide comprising a first extracellular antigen binding domain that binds a first antigen, an intracellular CD3ζ signaling domain, and a co-stimulatory polypeptide sequence from CD28, OX40 and 4-1BB; and b) a second polypeptide comprising a second extracellular antigen binding domain that binds a second antigen, and a T cell survival motif, wherein said second polypeptide does not comprise a co-stimulatory motif; wherein said modified lymphocyte survives only when said signaling domain and said T cell survival motif are both activated by said first antigen and said second antigen, respectively.

In another configuration, provided herein are modified T lymphocytes comprising: a) a first polypeptide comprising a first extracellular antigen binding domain that binds a first antigen, wherein said first antigen is a TAA or TSA, an intracellular CD3ζ signaling domain, and a co-stimulatory polypeptide sequence from CD27, CD28, OX40 (CD134), 4-1BB (CD137), or ICOS; and b) a second polypeptide comprising a second extracellular antigen binding domain that binds a second antigen, and a T cell survival motif, wherein said second polypeptide does not comprise a co-stimulatory motif; wherein said modified lymphocyte survives only when said signaling domain and said T cell survival motif are both activated by said first antigen and said second antigen, respectively.

In another configuration, provided herein are modified T lymphocytes comprising: a) a first polypeptide comprising a first extracellular antigen binding domain that binds a first antigen, wherein said first antigen is a TAA or TSA, an intracellular CD3ζ signaling domain, and a co-stimulatory polypeptide sequence from CD27, CD28, OX40 (CD134), 4-1BB (CD137), or ICOS; and b) a second polypeptide comprising a second extracellular antigen binding domain that binds a second antigen, wherein said second antigen is VEGF, bFGF, PDGF, HGF, IGF, or IL-8, and a T cell survival motif, wherein said second polypeptide does not comprise a co-stimulatory motif; wherein said modified lymphocyte survives only when said signaling domain and said T cell survival motif are both activated by said first antigen and said second antigen, respectively.

In another configuration, provided herein are modified T lymphocytes comprising: a) a first polypeptide comprising a first extracellular antigen binding domain that binds a first antigen, wherein said first antigen is Her2, PSCA, PSMA, BCMA, ERK5, AFP, CEA, CA-125, CA19-9, calretinin, MUC-1, EMA, ETA, tyrosinase, MAGE, CD34, CD45, CD99, CD117, chromogranin, cytokeratin, desmin, GFAP, GCDFP-15, HMB-45 antigen, MART-1, myo-D1, MSA, neurofilament, NSE, placental alkaline phosphatase, synaptophysin, thyroglobulin, thyroid transcription factor-1, tumor M2-PK, CD19, CD22, CD27, CD30, CD70, GD2 (ganglioside G2), EGFRvIII (epidermal growth factor variant III), sperm protein 17 (Sp17), mesothelin, PAP (prostatic acid phosphatase), prostein, TARP (T cell receptor gamma alternate reading frame protein), Trp-p8, STEAP1 (six-transmembrane epithelial antigen of the prostate 1), an abnormal ras protein, or an abnormal p53 protein; an intracellular CD3ζ signaling domain; and a co-stimulatory polypeptide sequence from CD27, CD28, OX40 (CD134), 4-1BB (CD137), or ICOS; and b) a second polypeptide comprising a second extracellular antigen binding domain that binds a second antigen, and a T cell survival motif, wherein said second polypeptide does not comprise a co-stimulatory motif; wherein said modified lymphocyte survives only when said signaling domain and said T cell survival motif are both activated by said first antigen and said second antigen, respectively.

In another configuration, provided herein are modified T lymphocytes comprising: a) a first polypeptide comprising a first extracellular antigen binding domain that binds a first antigen, wherein said first antigen is Her2, PSCA, PSMA, BCMA, ERK5, AFP, CEA, CA-125, CA19-9, calretinin, MUC-1, EMA, ETA, tyrosinase, MAGE, CD34, CD45, CD99, CD117, chromogranin, cytokeratin, desmin, GFAP, GCDFP-15, HMB-45 antigen, MART-1, myo-D1, MSA, neurofilament, NSE, placental alkaline phosphatase, synaptophysin, thyroglobulin, thyroid transcription factor-1, tumor M2-PK, CD19, CD22, CD27, CD30, CD70, GD2 (ganglioside G2), EGFRvIII (epidermal growth factor variant III), sperm protein 17 (Sp17), mesothelin, PAP (prostatic acid phosphatase), prostein, TARP (T cell receptor gamma alternate reading frame protein), Trp-p8, STEAP1 (six-transmembrane epithelial antigen of the prostate 1), an abnormal ras protein, or an abnormal p53 protein; an intracellular CD3ζ signaling domain; and a co-stimulatory polypeptide sequence from each of CD27, CD28, OX40 and 4-1BB; and b) a second polypeptide comprising a second extracellular antigen binding domain that binds a second antigen, wherein said second antigen is VEGF, bFGF, PDGF, HGF, IGF, or IL-8, and a T cell survival motif, wherein said second polypeptide does not comprise a co-stimulatory motif; wherein said modified lymphocyte survives only when said signaling domain and said T cell survival motif are both activated by said first antigen and said second antigen, respectively.

In another configuration, provided herein are modified T lymphocytes comprising: a) a first polypeptide comprising a first extracellular antigen binding domain that binds Her2, PSCA, PSMA, BCMA, ERK5, AFP, CEA, CA-125, CA19-9, calretinin, MUC-1, EMA, ETA, tyrosinase, MAGE, CD34, CD45, CD99, CD117, chromogranin, cytokeratin, desmin, GFAP, GCDFP-15, HMB-45 antigen, MART-1, myo-D1, MSA, neurofilament, NSE, placental alkaline phosphatase, synaptophysin, thyroglobulin, thyroid transcription factor-1, tumor M2-PK, CD19, CD22, CD27, CD30, CD70, GD2 (ganglioside G2), EGFRvIII (epidermal growth factor variant III), sperm protein 17 (Sp17), mesothelin, PAP (prostatic acid phosphatase), prostein, TARP (T cell receptor gamma alternate reading frame protein), Trp-p8, STEAP1 (six-transmembrane epithelial antigen of the prostate 1), an abnormal ras protein, or an abnormal p53 protein; an intracellular CD3ζ signaling domain; and a co-stimulatory polypeptide sequence from each of CD27, CD28, OX40 and 4-1BB; and b) a second polypeptide comprising a second extracellular antigen binding domain that binds VEGF, wherein said second polypeptide does not comprise a co-stimulatory motif; wherein said modified lymphocyte survives only when said signaling domain and said T cell survival motif are both activated by said first antigen and said second antigen, respectively.

### 4.1.8. Fifth Configuration, Basic Structure

In a fifth configuration, the two chimeric receptors contained within the modified T lymphocytes can be constructed so that a first chimeric receptor directed to a first antigen comprises a first extracellular antigen-binding domain and an T cell survival motif, e.g., an intracellular T cell survival motif, but no primary antigen binding signaling domain (e.g., CD3ζ), and no co-stimulatory domains, while a second chimeric receptor, directed to a second antigen, comprises a polypeptide sequence comprising a primary antigen binding signaling domain (e.g., CD3ζ), and one or more co-stimulatory domains. In this configuration, T lymphocytes are directed to cells expressing a desired antigen, and, upon antigen binding, a T lymphocyte survival signal is generated; however, in the absence of the binding of the second chimeric receptor to a second antigen, because no antigen binding and co-stimulatory signals are generated, T lymphocytes are not activated. As such, off-tumor effects are, again, eliminated or mitigated.

Thus, provided herein is modified T lymphocyte comprising: a) a first polypeptide comprising a first extracellular antigen binding domain that binds a first antigen, and a T cell survival motif, wherein said first polypeptide does not comprise a primary antigen binding signaling domain or a co-stimulatory motif; and b) a second polypeptide comprising a second extracellular antigen binding domain that binds a second antigen, and one or more co-stimulatory motifs; wherein said modified lymphocyte survives and is activated only when said first signaling domain and said second signaling domain are both activated by said first antigen and said second antigen, respectively. In certain embodiments, said T cell survival motif is an IL-7 receptor intracellular T cell survival motif is a T cell survival motif from CD28, IL-7R, IL-12R, IL-15R, IL-21R, or TGFßR.In certain embodiments, either or both of said first polypeptide or said second polypeptide comprise a transmembrane domain. In a more specific embodiment, said first polypeptide comprises a domain of CD27, CD28, IL-7R, IL-12R, IL-15R, IL-21R, or TGFßR that comprises a T cell survival motif.

As above, structurally the first antigen binding domain and said second antigen binding domain are independently any antigen binding domain, e.g., any of the types of antigen binding domains disclosed in Section 5.2, above, e.g., an antigen-binding portion of a receptor or an antigen-binding portion of an antibody. In a more specific embodiment, either or both of said first antigen binding domain or said second antigen binding domain are scFv antibody fragments. The first antigen may be any antigen of interest, e.g., an antigen that is expressed on the surface of a cell. In preferred embodiments, said first antigen is an antigen on a tumor cell. The tumor cell can be a cell, e.g., of a solid tumor or a blood cancer, e.g., any of the cancer or tumor types disclosed in Section 5.1, above. In certain specific embodiments, said antigen is a TAA or TSA, e.g., any of the TAAs or TSAs disclosed in Section 5.1, above. The second antigen, which is different from the first antigen, can be an angiogenic or vasculogenic factor, e.g., any of the angiogenic or vasculogenic factors disclosed in Section 5.1, above; or any DAMP, e.g., the DAMPs disclosed in Section 5.1, above. In other more specific embodiments, signal transduction by said second chimeric receptor is induced by activation of a hypoxia-associated factor, e.g., HIF-1α, HIF-1β, HIF-2α, HIF-2β, HIF-3α, or HIF-3β.

The intracellular signaling domain of the second polypeptide can be any polypeptide that is capable of transmitting an antigen binding signal from the antigen binding domain of the second polypeptide, e.g., in a manner similar to the CD3ζ (CD3 zeta) chains of the native T lymphocyte receptor. In certain embodiments, the intracellular signaling domain comprises a polypeptide sequence comprising an immunoreceptor tyrosine-based activation motif (ITAM). Preferably, the polypeptide sequence is a CD3ζ signaling domain or a signal-transducing variant thereof. The second polypeptide additionally comprises one or more co-stimulatory domains, e.g., any co-stimulatory motif or functional portion thereof, e.g., a co-stimulatory CD27, CD28, OX40 (CD134), 4-1BB (CD137), or ICOS polypeptide sequence.

### 4.1.9. Specific Embodiments

In one configuration, provided herein are modified T lymphocytes comprising: a) a first polypeptide comprising a first extracellular antigen binding domain that binds a first antigen, and a T cell survival motif, wherein said first polypeptide does not comprise a primary antigen binding signaling domain and does not comprise a co-stimulatory motif; and b) a second polypeptide comprising a second extracellular antigen binding domain that binds a second antigen, an intracellular CD3ζ signaling domain, and one or more co-stimulatory motifs; wherein said modified lymphocyte is activated and survives only when said signaling domain and said T cell survival motif are activated by said second antigen and said first antigen, respectively.

In another configuration, provided herein are modified T lymphocytes comprising: a) a first polypeptide comprising a first extracellular antigen binding domain that binds a first antigen, and a T cell survival motif, wherein said first polypeptide does not comprise a primary antigen binding signaling domain and does not comprise a co-stimulatory motif; and b) a second polypeptide comprising a second extracellular antigen binding domain that binds a second antigen, an intracellular CD3ζ signaling domain, and a co-stimulatory polypeptide sequence from CD27, CD28, OX40 (CD134), 4-1BB (CD137), or ICOS; wherein said modified lymphocyte is activated and survives only when said T cell survival motif and said signaling domain are activated by said first antigen and said second antigen, respectively.

In another configuration, provided herein are modified T lymphocytes comprising: a) a first polypeptide comprising a first extracellular antigen binding domain that binds a first antigen, and a T cell survival motif, wherein said first polypeptide does not comprise a primary antigen binding signaling domain and does not comprise a co-stimulatory motif; and b) a second polypeptide comprising a second extracellular antigen binding domain that binds a second antigen, wherein said second antigen is an angiogenic or vasculogenic factor, or a DAMP, an intracellular CD3ζ signaling domain, and a co-stimulatory polypeptide sequence from CD27, CD28, OX40 (CD134), 4-1BB (CD137), or ICOS; wherein said second polypeptide does not comprise a primary antigen binding signaling domain and does not comprise a co-stimulatory motif; wherein said modified lymphocyte survives only when said T cell survival motif and said signaling domain are both activated by said first antigen and said second antigen, respectively.

In another configuration, provided herein are modified T lymphocytes comprising: a) a first polypeptide comprising a first extracellular antigen binding domain that binds a first antigen, and a T cell survival motif, wherein said first polypeptide does not comprise a primary antigen binding signaling domain and does not comprise a co-stimulatory motif; and b) a second polypeptide comprising a second extracellular antigen binding domain that binds a second antigen, wherein said second antigen is VEGF, bFGF, PDGF, HGF, IGF, or IL-8, an intracellular CD3ζ signaling domain, and a co-stimulatory polypeptide sequence from CD27, CD28, OX40 (CD134), 4-1BB (CD137), or ICOS; wherein said modified lymphocyte survives only when said T cell survival motif said signaling domain are both activated by said first antigen and said second antigen, respectively.

In another configuration, provided herein are modified T lymphocytes comprising: a) a first polypeptide comprising a first extracellular antigen binding domain that binds a first antigen, and a T cell survival motif, wherein said first polypeptide does not comprise a primary antigen binding signaling domain and does not comprise a co-stimulatory motif; and b) a second polypeptide comprising a second extracellular antigen binding domain that binds a second antigen, an intracellular CD3ζ signaling domain, and a co-stimulatory polypeptide sequence from CD28, OX40 and 4-1BB; wherein said modified lymphocyte survives only when said T cell survival motif and said signaling domain are both activated by said first antigen and said second antigen, respectively.

In another configuration, provided herein are modified T lymphocytes comprising: a) a first polypeptide comprising a first extracellular antigen binding domain that binds a first antigen, wherein said first antigen is a TAA or TSA, and a T cell survival motif, wherein said first polypeptide does not comprise a primary antigen binding signaling domain and does not comprise a co-stimulatory motif; and b) a second polypeptide comprising a second extracellular antigen binding domain that binds a second antigen, an intracellular CD3ζ signaling domain, and a co-stimulatory polypeptide sequence from CD27, CD28, OX40 (CD134), 4-1BB (CD137), or ICOS; wherein said modified lymphocyte survives only when said T cell survival motif and said signaling domain are both activated by said first antigen and said second antigen, respectively.

In another configuration, provided herein are modified T lymphocytes comprising: a) a first polypeptide comprising a first extracellular antigen binding domain that binds a first antigen, wherein said first antigen is a TAA or TSA, and a T cell survival motif, wherein said first polypeptide does not comprise a primary antigen binding signaling domain and does not comprise a co-stimulatory motif; and b) a second polypeptide comprising a second extracellular antigen binding domain that binds VEGF, bFGF, PDGF, HGF, IGF, or IL-8, an intracellular CD3ζ signaling domain, and a co-stimulatory polypeptide sequence from CD27, CD28, OX40 (CD134), 4-1BB (CD137), or ICOS; wherein said modified lymphocyte survives only when said T cell survival motif and said signaling domain are both activated by said first antigen and said second antigen, respectively.

In another configuration, provided herein are modified T lymphocytes comprising: a) a first polypeptide comprising a first extracellular antigen binding domain that binds a first antigen, wherein said first antigen is Her2, PSCA, PSMA, BCMA, ERK5, AFP, CEA, CA-125, CA19-9, calretinin, MUC-1, EMA, ETA, tyrosinase, MAGE, CD34, CD45, CD99, CD117, chromogranin, cytokeratin, desmin, GFAP, GCDFP-15, HMB-45 antigen, MART-1, myo-D1, MSA, neurofilament, NSE, placental alkaline phosphatase, synaptophysin, thyroglobulin, thyroid transcription factor-1, tumor M2-PK, CD19, CD22, CD27, CD30, CD70, GD2 (ganglioside G2), EGFRvIII (epidermal growth factor variant III), sperm protein 17 (Sp17), mesothelin, PAP (prostatic acid phosphatase), prostein, TARP (T cell receptor gamma alternate reading frame protein), Trp-p8, STEAP1 (six-transmembrane epithelial antigen of the prostate 1), an abnormal ras protein, or an abnormal p53 protein; and a T cell survival motif, wherein said first polypeptide does not comprise a primary antigen binding signaling domain and does not comprise a co-stimulatory motif; and b) a second polypeptide comprising a second extracellular antigen binding domain that binds a second antigen, an intracellular CD3ζ signaling domain; and a co-stimulatory polypeptide sequence from CD27, CD28, OX40 (CD134), 4-1BB (CD137), or ICOS; wherein said modified lymphocyte survives only when said T cell survival motif and said signaling domain are both activated by said first antigen and said second antigen, respectively.

In another configuration, provided herein are modified T lymphocytes comprising: a) a first polypeptide comprising a first extracellular antigen binding domain that binds a first antigen, wherein said first antigen is Her2, PSCA, PSMA, BCMA, ERK5, AFP, CEA, CA-125, CA19-9, calretinin, MUC-1, EMA, ETA, tyrosinase, MAGE, CD34, CD45, CD99, CD117, chromogranin, cytokeratin, desmin, GFAP, GCDFP-15, HMB-45 antigen, MART-1, myo-D1, MSA, neurofilament, NSE, placental alkaline phosphatase, synaptophysin, thyroglobulin, thyroid transcription factor-1, tumor M2-PK, CD19, CD22, CD27, CD30, CD70, GD2 (ganglioside G2), EGFRvIII (epidermal growth factor variant III), sperm protein 17 (Sp17), mesothelin, PAP (prostatic acid phosphatase), prostein, TARP (T cell receptor gamma alternate reading frame protein), Trp-p8, STEAP1 (six-transmembrane epithelial antigen of the prostate 1), an abnormal ras protein, or an abnormal p53 protein; and a T cell survival motif, wherein said first polypeptide does not comprise a primary antigen binding signaling domain and does not comprise a co-stimulatory motif; and b) a second polypeptide comprising a second extracellular antigen binding domain that binds a second antigen, wherein said second antigen is VEGF, bFGF, PDGF, HGF, IGF, or IL-8; an intracellular CD3ζ signaling domain; and a co-stimulatory polypeptide sequence from each of CD27, CD28, OX40 and 4-1BB; wherein said modified lymphocyte survives only when said T cell survival motif and said signaling domain are both activated by said first antigen and said second antigen, respectively.

In another configuration, provided herein are modified T lymphocytes comprising: a) a first polypeptide comprising a first extracellular antigen binding domain that binds Her2, PSCA, PSMA, BCMA, ERK5, AFP, CEA, CA-125, CA19-9, calretinin, MUC-1, EMA, ETA, tyrosinase, MAGE, CD34, CD45, CD99, CD117, chromogranin, cytokeratin, desmin, GFAP, GCDFP-15, HMB-45 antigen, MART-1, myo-D1, MSA, neurofilament, NSE, placental alkaline phosphatase, synaptophysin, thyroglobulin, thyroid transcription factor-1, tumor M2-PK, CD19, CD22, CD27, CD30, CD70, GD2 (ganglioside G2), EGFRvIII (epidermal growth factor variant III), sperm protein 17 (Sp17), mesothelin, PAP (prostatic acid phosphatase), prostein, TARP (T cell receptor gamma alternate reading frame protein), Trp-p8, STEAP1 (six-transmembrane epithelial antigen of the prostate 1), an abnormal ras protein, or an abnormal p53 protein; and a T cell survival motif, wherein said first polypeptide does not comprise a primary antigen binding signaling domain and does not comprise a co-stimulatory motif; and b) a second polypeptide comprising a second extracellular antigen binding domain that binds a second antigen, wherein said second antigen is VEGF; an intracellular CD3ζ signaling domain; and a co-stimulatory polypeptide sequence from each of CD27, CD28, OX40 and 4-1BB; wherein said modified lymphocyte survives only when said T cell survival motif and said signaling domain are both activated by said first antigen and said second antigen, respectively.

### 4.1.10. Other Configurations

In certain embodiments, the modified T lymphocyte comprises a modified TCR as a first polypeptide, and an artificial second polypeptide that produces a co-stimulatory signal. In specific embodiments, the modified TCR is modified, e.g., by replacement of the native antigen-binding domain with a domain that binds to a specific antigen. In a specific embodiment, the T lymphocytes are transformed with polynucleotides encoding alpha and beta chains of the anti-MART-1 TCR. T lymphocytes may similarly be transformed with polynucleotides encoding alpha and beta TCR subunits, where the TCR is directed to an antigen, e.g., a TSA or TAA. In preferred embodiments, the modified T lymphocyte is additionally transformed with a polynucleotide that encodes an artificial co-stimulatory polypeptide, e.g., the co-stimulatory polypeptide disclosed in Section 5.1, above, or one of the second polypeptides disclosed in Section 5.2, above.

In certain embodiments in which modified T lymphocytes comprise two polypeptides, e.g., chimeric receptors, a first polypeptide comprises a first antigen binding domain, a primary antigen binding signal transduction domain (e.g., CD3ζ), and a single co-stimulatory domain (e.g., CD28 or a co-stimulatory polypeptide sequence therefrom), and a second polypeptide that comprises a second antigen binding domain and at least one co-stimulatory domain, e.g., a co-stimulatory domain from CD27, 4-1BB, OX40, IL-7R or the like. In a more specific embodiment, the second polypeptide comprises at least two, or at least three co-stimulatory domains.

In certain other embodiments, the modified T lymphocytes provided herein comprise a first polypeptide (e.g., chimeric receptor) comprising a first antigen binding domain, a primary antigen binding signal transduction domain (e.g., CD3ζ), and no co-stimulatory domain (e.g., CD28 or a co-stimulatory polypeptide sequence therefrom); a second polypeptide (chimeric receptor) comprising a second antigen-binding domain and at least one co-stimulatory domain; and a third polypeptide comprising an antigen-binding domain and at least one other co-stimulatory domain. In this embodiment, the total number of co-stimulatory domains is divided between at least two separate chimeric receptors. The at least two different chimeric receptors can comprise antigen binding domains that bind to the same antigen, or different antigens.

### 4.2. Isolated Polypeptides (Chimeric Antigen Receptors)

The first and second polypeptides provided herein, useful for producing the modified T lymphocytes provided herein, may be modified by, e.g., acylation, amidation, glycosylation, methylation, phosphorylation, sulfation, sumoylation, ubiquitylation, or the like. The polypeptides may be labeled with a label capable of providing a detectable signal, e.g., with radioisotopes and fluorescent compounds. One or more side chains of the first or second polypeptides may be derivatized, e.g., derivatization of lysinyl and amino terminal residues with succinic or other carboxylic acid anhydrides, or derivatization with, e.g., imidoesters such as methyl picolinimidate; pyridoxal phosphate; pyridoxal; chloroborohydride; trinitrobenzenesulfonic acid; O-methylisourea; 2,4 pentanedione; and transaminase-catalyzed reaction with glyoxylate. Carboxyl side groups, aspartyl or glutamyl, may be selectively modified by reaction with carbodiimides (R-N=C=N-R') such as 1-cyclohexyl-3-(2-morpholinyl-(4-ethyl)carbodiimide or 1-ethyl-3-(4-azonia-4,4-dimethylpentyl)carbodiimide.

### 4.3. Isolated Nucleic Acids

The disclosed polypeptides (e.g., chimeric receptors) can be encoded by polynucleotide sequences according to well-known methods in the art. The polynucleotides may be contained within any polynucleotide vector suitable for the transformation of immune cells, e.g., T lymphocytes. For example, T lymphocytes may be transformed using synthetic vectors, lentiviral or retroviral vectors, autonomously replicating plasmids, a virus (e.g., a retrovirus, lentivirus, adenovirus, or herpes virus), or the like, containing polynucleotides encoding the first and second polypeptides (e.g., chimeric receptors). Lentiviral vectors suitable for transformation of T lymphocytes include, but are not limited to, e.g., the lentiviral vectors described in U.S. Patent Nos. 5,994,136; 6,165,782; 6,428,953; 7,083,981; and 7,250,299. HIV vectors suitable for transformation of T lymphocytes include, but are not limited to, e.g., the lentiviral vectors described in U.S. Patent No. 5,665,577.

Nucleic acids useful in the production of the first and second polypeptides, e.g., within a modified T lymphocyte, include DNA, RNA, or nucleic acid analogs. Nucleic acid analogs can be modified at the base moiety, sugar moiety, or phosphate backbone, and can include deoxyuridine substitution for deoxythymidine, 5-methyl-2'-deoxycytidine or 5-bromo-2'-deoxycytidine substitution for deoxycytidine. Modifications of the sugar moiety can include modification of the 2' hydroxyl of the ribose sugar to form 2'-O-methyl or 2'-O-allyl sugars. The deoxyribose phosphate backbone can be modified to produce morpholino nucleic acids, in which each base moiety is linked to a six membered, morpholino ring, or peptide nucleic acids, in which the deoxyphosphate backbone is replaced by a pseudopeptide backbone and the four bases are retained. See, for example, Summerton and Weller (1997) Antisense Nucleic Acid Drug Dev. 7:187-195; and Hyrup et al. (1996) Bioorgan. Med. Chain. 4:5-23. In addition, the deoxyphosphate backbone can be replaced with, for example, a phosphorothioate or phosphorodithioate backbone, a phosphoroamidite, or an alkyl phosphotriester backbone.

### 4.4. T Lymphocytes

The T lymphocytes used in the compositions and methods provided herein may be naive T lymphocytes or MHC-restricted T lymphocytes. In certain embodiments, the T lymphocytes are tumor infiltrating lymphocytes (TILs). In certain embodiments, the T lymphocytes have been isolated from a tumor biopsy, or have been expanded from T lymphocytes isolated from a tumor biopsy. In certain other embodiments, the T cells have been isolated from, or are expanded from T lymphocytes expanded from, peripheral blood, cord blood, or lymph.

The immune cells, e.g., modified T lymphocytes, used in the present methods are preferably autologous to an individual to whom the modified T lymphocytes are to be administered. In certain other embodiments, the modified T lymphocytes are allogeneic to an individual to whom the modified T lymphocytes are to be administered. Where allogeneic T lymphocytes are used to prepare modified T lymphocytes, it is preferable to select T lymphocytes that will reduce the possibility of graft-versus-host disease (GVHD) in the individual. For example, in certain embodiments, virus-specific T lymphocytes are selected for preparation of modified T lymphocytes; such lymphocytes will be expected to have a greatly reduced native capacity to bind to, and thus become activated by, any recipient antigens. In certain embodiments, recipient-mediated rejection of allogeneic T lymphocytes can be reduced by co-administration to the host of one or more immunosuppressive agents, e.g., cyclosporine, tacrolimus, sirolimus, cyclophosphamide, or the like.

In one embodiment, T lymphocytes are obtained from an individual, optionally then expanded, and then transformed with a first polynucleotide encoding the first polypeptide and a second polynucleotide encoding the second polypeptide, and optionally then expanded. Double transformants may be selected using, e.g., a selectable marker unique to each of the vectors. In another embodiment, T lymphocytes are obtained from an individual, optionally then expanded, and then transformed with a polynucleotide encoding the first polypeptide and the second polypeptide, and optionally then expanding. Cells containing the polynucleotide are selected using a selectable marker.

In certain embodiments, the modified T lymphocytes comprise native TCR proteins, e.g., TCR-α and TCR-β that are capable of forming native TCR complexes, in addition to the artificial co-stimulatory polypeptide (in embodiments in which a single co-stimulatory polypeptide is used), or in addition to the first polypeptide and second polypeptide (in embodiments in which the modified T lymphocytes comprise polypeptides separating the antigen binding signaling and co-stimulatory signaling). In certain other embodiments, either or both of the native genes encoding TCR-α and TCR-β in the modified T lymphocytes are modified to be non-functional, e.g., a portion or all are deleted, a mutation is inserted, etc.

In certain embodiments, the T lymphocytes are isolated from a tumor lesion, e.g., are tumor-infiltrating lymphocytes; such T lymphocytes are expected to be specific for a TSA or TAA.

In certain embodiments, the signaling motifs of the first polypeptide and the second polypeptide can be used to promote proliferation and expansion of the modified T lymphocytes. For example, unmodified T lymphocytes, and T lymphocytes comprising a polypeptide comprising a CD3ζ signaling domain and a CD28 co-stimulatory domain can be expanded using antibodies to CD3 and CD28, e.g., antibodies attached to beads; see, e.g., U.S. Patent Nos. 5,948,893; 6,534,055; 6,352,694; 6,692,964; 6,887,466; and 6,905,681. Similarly, antibodies to a signaling motif on the first polypeptide and antibodies to a signaling motif on the second polypeptide can be used to stimulate proliferation of T lymphocytes comprising both the first and second polypeptides.

In certain embodiments, whether the first and second polypeptides are expressed with the T lymphocyte from a single vector or two separate vectors, the first and second antigens to which the first polypeptide and second polypeptide bind, respectively, can be used to promote selective expansion of T lymphocytes expressing both the first polypeptide and the second polypeptide. For example, in one embodiment, in which the first antigen, to which the first polypeptide binds, is a TSA, and the second antigen, to which the second polypeptide binds, is an angiogenic factor, the T lymphocytes comprising the first polypeptide and second polypeptide are cultured in the presence of the TSA and the angiogenic factor, resulting in increased proliferation as compared to culturing in the presence of the first or second antigens, alone, or in the absence of either.

In certain other embodiments, The T lymphocytes comprising the first and second polypeptides are stimulated to proliferate using an antibody that binds to a signaling domain on the first polypeptide coupled with an antigen that can be bound by the second polypeptide. For example, in embodiments in which the first polypeptide's signaling domain is CD3ζ and the antigen that binds to the second polypeptide is VEGF, T lymphocytes comprising the first and second polypeptides are stimulated to proliferate by culturing the cells in the presence of VEGF in combination with an antibody that binds to CD3ζ. In other embodiments, the T lymphocytes comprising the first and second polypeptides are stimulated to proliferate using an antigen that can be bound by the first polypeptide and a co-stimulatory motif on the second peptide. For example, in embodiments in which the antigen that binds to the first polypeptide is HER2 and the co-stimulatory motif on the second polypeptide is obtained from CD28, T lymphocytes comprising the first and second polypeptides are stimulated to proliferate by culturing the cells in the presence of HER2 protein and an antibody that binds to CD28.

In any of the above embodiments, the antigen and/or antibody can exist free in the medium in which the T lymphocytes are cultures, or either or both can be attached to a solid support, e.g., tissue culture plastic surface, beads, or the like.

The modified T lymphocytes can optionally comprise a "suicide gene" or "safety switch" that enables killing of substantially all of the modified T lymphocytes when desired. For example, the modified T lymphocytes, in certain embodiments, can comprise an HSV thymidine kinase gene (HSV-TK), which causes death of the modified T lymphocytes upon contact with gancyclovir. In another embodiment, the modified T lymphocytes comprise an inducible caspase, e.g., an inducible caspase 9 (icaspase9), e.g., a fusion protein between caspase 9 and human FK506 binding protein allowing for dimerization using a specific small molecule pharmaceutical. See Straathofet al., Blood 105(11):4247-4254 (2005).

### 4.5. Methods of Using Modified T Lymphocytes

The modified immune cells, e.g., the modified T lymphocytes provided herein, can be used to treat an individual having one or more types of cells desired to be targeted by T lymphocytes, e.g., to be killed. In certain embodiments, the cells to be killed are cancer cells, e.g., tumor cells. In preferred embodiments, the cancer cells are cells of a solid tumor. In specific embodiments, the cells are cells of a lymphoma, a lung cancer, a breast cancer, a prostate cancer, an adrenocortical carcinoma, a thyroid carcinoma, a nasopharyngeal carcinoma, a melanoma, e.g., a malignant melanoma, a skin carcinoma, a colorectal carcinoma, a desmoid tumor, a desmoplastic small round cell tumor, an endocrine tumor, an Ewing sarcoma, a peripheral primitive neuroectodermal tumor, a solid germ cell tumor, a hepatoblastoma, a neuroblastoma, a non-rhabdomyosarcoma soft tissue sarcoma, an osteosarcoma, a retinoblastoma, a rhabdomyosarcoma, a Wilms tumor, a glioblastoma, a myxoma, a fibroma, a lipoma, or the like. In more specific embodiments, said lymphoma can be chronic lymphocytic leukemia (small lymphocytic lymphoma), B-cell prolymphocytic leukemia, lymphoplasmacytic lymphoma, Waldenstrom macroglobulinemia, splenic marginal zone lymphoma, plasma cell myeloma, plasmacytoma, extranodal marginal zone B cell lymphoma, MALT lymphoma, nodal marginal zone B cell lymphoma, follicular lymphoma, mantle cell lymphoma, diffuse large B cell lymphoma, mediastinal (thymic) large B cell lymphoma, intravascular large B cell lymphoma, primary effusion lymphoma, Burkitt's lymphoma, T lymphocyte prolymphocytic leukemia, T lymphocyte large granular lymphocytic leukemia, aggressive NK cell leukemia, adult T lymphocyte leukemia/lymphoma, extranodal NK/T lymphocyte lymphoma, nasal type, enteropathy-type T lymphocyte lymphoma, hepatosplenic T lymphocyte lymphoma, blastic NK cell lymphoma, mycosis fungoides, Sezary syndrome, primary cutaneous anaplastic large cell lymphoma, lymphomatoid papulosis, angioimmunoblastic T lymphocyte lymphoma, peripheral T lymphocyte lymphoma (unspecified), anaplastic large cell lymphoma, Hodgkin lymphoma, or a non-Hodgkin lymphoma.

Efficacy of the modified T lymphocytes, after administration to an individual having a disease or disorder remediable by T lymphocytes, e.g., an individual having cancer, can be assessed by one or more criteria, specific to the particular disease or disorder, known to those of ordinary skill in the art, to be indicative of progress of the disease or disorder. Generally, administration of the modified T lymphocytes to such an individual is effective when one or more of said criteria detectably, e.g., significantly, moves from a disease state value or range to, or towards, a normal value or range.

The modified T lymphocytes may be formulated in any pharmaceutically-acceptable solution, preferably a solution suitable for the delivery of living cells, e.g., saline solution (such as Ringer's solution), gelatins, carbohydrates (e.g., lactose, amylose, starch, or the like), fatty acid esters, hydroxymethylcellulose, polyvinyl pyrolidine, etc. Such preparations are preferably sterilized prior to addition of the modified T lymphocytes, and may be mixed with auxiliary agents such as lubricants, preservatives, stabilizers, emulsifiers, salts for influencing osmotic pressure, buffers, and coloring. Pharmaceutical carriers suitable for use in formulating the modified T lymphocytes are known in the art and are described, for example, in WO 96/05309.

In certain embodiments, the modified T lymphocytes are formulated into individual doses, wherein said individual doses comprise at least, at most, or about 1×10⁴, 5×10⁴, 1×10⁵, 5×10⁵, 1×10⁶, 5×10⁶, 1×10⁷, 5×10⁷, 1×10⁸, 5×10⁸, 1×10⁹, 5×10⁹, 1×10¹⁰, 5×10¹⁰, or 1×10¹¹ modified T lymphocytes. In certain embodiments, the modified T lymphocytes are formulated for intravenous, intraarterial, parenteral, intramuscular, subcutaneous, intrathecal, or intraocular administration, or administration within a particular organ or tissue.

### 5. EXAMPLES

### 5.1. Example 1: Treatment of Prostate Cancer

An individual presents with stage T2 prostate cancer, with no spread to regional or other lymph nodes (N0, M0). Histological grade is determined to be G2. Overall, the individual is determined to have Stage II prostate cancer. The individual is administered between 10⁹ and 10¹⁰ modified T lymphocytes that comprise a single chimeric receptor, in 200 mL saline solution by intravenous infusion over 30 minutes. The chimeric receptor comprises an extracellular antigen-binding region that binds to PSCA, a transmembrane domain, and intracellular co-stimulatory domains from each of CD27, CD28, 4-1BB, and OX40. The individual is re-assessed for prostate cancer stage and spread to lymph nodes, and histology of biopsied prostate tissue is performed, at 30, 60 and 90 days post-administration.

### 5.2. Example 2: Treatment of Prostate Cancer

An individual presents with stage T2 prostate cancer, with no spread to regional or other lymph nodes (N0, M0). Histological grade is determined to be G2. Overall, the individual is determined to have Stage II prostate cancer. The individual is administered between 10⁹ and 10¹⁰ modified T lymphocytes that comprise a first and second chimeric receptor, in 200 mL saline solution by intravenous infusion over 30 minutes. The first chimeric receptor comprises an extracellular antigen-binding region that binds to PSCA, a transmembrane domain, and a signal transduction domain derived from CD3ζ. The second chimeric receptor comprises an antigen-binding domain that binds to the protein ERK5, a transmembrane domain, and intracellular co-stimulatory domains from each of CD27, CD28, 4-1BB, and OX40. The individual is re-assessed for prostate cancer stage and spread to lymph nodes, and histology of biopsied prostate tissue is performed, at 30, 60 and 90 days post-administration.

### 5.3. Example 3: Treatment of Breast Cancer

An individual presents with stage 3 breast cancer that has spread to at least one regional lymph node. After surgery to remove cancerous tissue, the individual is administered between 10⁹ and 10¹⁰ modified T lymphocytes that comprise a first and second chimeric receptor, in 200 mL saline solution by intravenous infusion over 30 minutes. The first chimeric receptor comprises an extracellular antigen-binding region that binds to HER2, a transmembrane domain, and a signal transduction domain derived from CD3ζ. The second chimeric receptor comprises an antigen-binding domain that binds to the estrogen receptor (ER), a transmembrane domain, and intracellular co-stimulatory domains from each of CD27, CD28, 4-1BB, and OX40. The individual is assessed for breast cancer in remaining breast tissue, and spread to other lymph nodes, 30, 60, 90 and 180 days post-administration.

### 5.4. Example 4: Modified T Lymphocytes That Have Dual Antigen Specificity

This Example describes the generation of modified T lymphocytes comprising two chimeric antigen receptors (CARs), wherein the first CAR comprises an antigen binding domain specific to a tumor-specific antigen and wherein the second CAR comprises an antigen binding domain specific to an antigen that is not a tumor-specific antigen, but that is associated with tumorigenesis.

### CAR Constructs

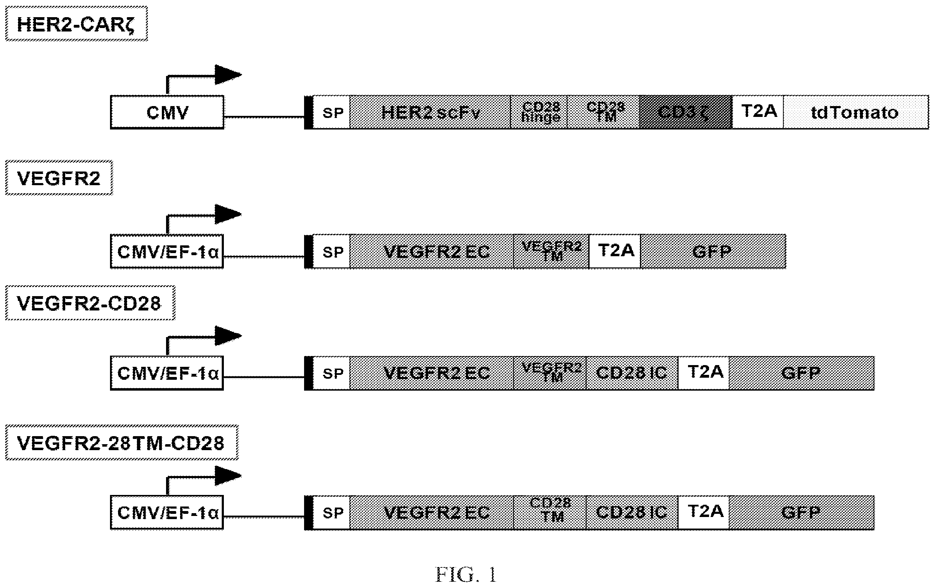

The CARs depicted in Figure 1 were prepared using standard methodology. An anti-HER2-based CAR, "HER2-CARζ," consisting of a scFv of an anti-HER2 antibody linked to a CD28 hinge, a CD28 transmembrane (TM) domain, a CD3 zeta chain, and a tdTomato reporter gene was constructed and cloned into a lentivirus vector. This CAR represents a CAR comprising an antigen binding domain specific to a tumor-specific antigen, and a stimulatory domain.

Two CARs, "VEGFR2-CD28" and "VEGFR2-28TM-CD28," comprising an antigen binding domain specific to VEGF, an antigen that is not a tumor-specific antigen, and a costimulatory domain, were generated. Both CARs comprise an antigen binding domain made up of a portion of a receptor for the VEGF antigen, namely, VEGFR2. VEGFR2-CD28 comprises the human VEGFR2 extracellular (EC) domain followed by a VEGFR2 TM domain, a CD28 IC domain, a T2A sequence (thosea asigna virus 2A peptide), and GFP (for use as a reporter gene). VEGFR2-28TM-CD28 comprises the human VEGFR2 extracellular (EC) domain followed by a CD28 TM domain, a CD28 intracellular (IC) domain, a T2A sequence, and GFP.

A control construct for VEGF recognition also was generated. The control construct, designated as "VEGFR2," comprises the human VEGFR2 extracellular (EC) domain followed by a VEGFR2 TM domain, a T2A sequence, and GFP. The control construct thus lacks the CD28 IC domain present in the constructs designated VEGFR2-CD28 and VEGFR2-28TM-CD28.

### Expression of CAR Constructs in T cells

The expression of the above-described CAR constructs by T cells was examined. To isolate T cells, peripheral blood mononuclear cells (PBMC) were separated from healthy donor whole blood-derived buffy coats using Ficoll-Paque Plus^{™} density gradient centrifugation (GE Healthcare, Piscataway, NJ). Pan T cells were negatively selected from PBMCs using the Pan T Isolation Kit II (Miltenyi Biotec, Cambridge, MA), according to the manufacturer's instructions.

Plasmids comprising the HER2-CARζ, VEGFR2-CD28, VEGFR2-28TM-CD28, or VEGFR2 CAR constructs were electroporated into primary T cells, and the electroporated T cells were cultured in RPMI-10 media overnight. T cells were harvested at 24 hours post electroproation and stained with HER2-human IgG-Fc chimera protein, followed by staining with a goat anti-human IgG-Fc polyclonal antibody conjugated with APC (for anti-HER2 detection); or a mouse anti-human VEGFR2 monoclonal antibody (mAb; for VEGFR2 detection). The stained cells were analyzed by flow cytometry. In all instances, expression of the antigen binding domain of the CARs and the reporter genes of each CAR (tdTomato or GFP) were detected, confirming stable transduction of T cells by the transgenes. Transduction of IL-7 activated T cells with each CAR construct further confirmed that the HER2-CARζ construct can mediate anti-HER2 expression in transfected T cells and that the VEGFR2-CD28, VEGFR2-28TM-CD28, and VEGFR2 CAR constructs can mediate positive VEGFR2 expression in transfected T cells.

### VEGF Production and VEGFR2 Expression by Activated T cells

To ensure that endogenous VEGFR2 expression does not compete with expression of the VEGFR2 extracellular domain in the VEGFR2 EC domain containing constructs for binding to the VEGF, preliminary experiments were carried out to assess levels of VEGF production and VEGFR2 expression by activated T cells.

Human primary T cells were isolated as described above and stimulated with anti-CD3/CD28 DynaBeads^{®} at a ratio of 3 to 1 beads per cell. The cells were cultured in RPMI-10 media in the presence of IL-2 at 50IU/ml. VEGFR2 expression by the stimulated T cells was assessed via flow cytometry for 25 days post stimulation (every day for the first 4 days, then every two days after the first week). VEGFR2 expression by activated T cells was not observed until day 2 post stimualtion, followed by a dramatic decrease in expression by day 3 and disappearance of expression by day 4. After day 4, VEGFR2 expression was not observed.

VEGF-A in the supernant of T cells after the DynaBead activation described above was measured via Cytometric Bead Array (CBA). Minimal VEGF secretion (<10pg/ml) was detected.

The data suggest that activated human T cells minimally express endogenous VEGF and VEGFR2.

### Costimulation Assay

To assess the ability of the constructs comprising the VEGFR2 EC domain to mediate costimulation, human primary pan T cells were transfected with either the VEGFR2-CD28, VEGFR2-28TM-CD28, or VEGFR2 lentivectors followed by stimulation with immobilized anti-human CD3 and anti-human VEGFR2 mAb or soluble VEGF. Anti-human CD3 was selected as a ligand to trigger the first "signal" in the dual signaling system (that is, binding of a tumor antigen by a CAR comprising an antigen binding domain specific to a tumor antigen and an activation domain (e.g., a CD3 zeta chain)). Culturing the T cells with either anti-VEGFR2 mAb or soluble VEGF resulted in stimulation of T cells that had been transfected with either VEGFR2-CD28 lentivector or VEGFR2-28TM-CD28 lentivector, but not T cells transfected with VEGFR2 lentivector, as evidenced by upregulation of the activation markers CD69 and 4-1BB.

In addition, it was determined that VEGFR2-CD28 lentivector and VEGFR2-28TM-CD28 lentivector transfected T cells, but not T cells transfected with VEGFR2 lentivector, secrete elevated levels of IL-2, granzyme B, and IFN-γ upon anti-VEGFR2 mAb treatment or VEGF treatment. Taken together, the results indicate that expression of the VEGFR2 EC domain in transfected T cells can mediate intracellular CD28 signaling when VEGFR2 EC is expressed as part of a CAR comprising a CD28 IC domain.

### Functional Evaluation of HER2-CARζ

Functional validation of HER2-CARζ was determined in transfected T cells by stimulation of the T cells with immobilized HER2-Fc chimera protein. As a positive control for CD28 costimulation, another construct, "HER2-CAR28ζ," which is identical to the CAR construct designated HER2-CARζ with the exception of the inclusion of a CD28 intracellular domain between the CD28 transmembrane (TM) domain and the CD3 zeta chain, was generated.

To determine stimulation of the T cells by HER2-Fc chimera protein, expression of the T cell activation markers CD69 and CD71 was examined 48 hours post-stimulation. Only tdTomato-positive cells showed CD69 and CD71 up-regulation in both HER2-CARζ and HER2-CAR28ζ transfected cells. Higher frequency and mean fluorescence intensity of CD69 and CD71 were observed in T cells transfected with HER2-CAR28ζ as compared to T cells transfected with with HER2-CARζ (42% HER2-CAR28ζ cells expressed CD69 as compared to 25% expression by HER2-CARζ cells (mock transfected cells = 0.04% expression); 27% HER2-CAR28ζ cells expressed CD71 as compared to 10% expression by HER2-CARζ cells (mock transfected cells = 0.04% expression)), indicating activity of intracellular CD28 signaling domain of the HER2-CAR28ζ construct.

The effect of culturing with anti-VEGFR2 mAb or VEGF with HER2-CARζ or HER2-CAR28ζ transfected T cells also was determined. T cells were treated with HER2-Fc and VEGFR2 mAb or VEGF for 48 hours, followed by flow cytometric analysis to assess surface expression of CD69 and CD71. Only very minimal enhancement of both CD69 and CD71 expression relative to that described above was observed.

### Evaluation of the Dual Signaling System

After VEGFR2-mediated costimulation was confirmed, HER2-CARζ and VEGFR2-CD28IC dual signaling was assessed. To assess dual signaling, T cells were isolated as described above and transfected with both (i) a CAR that comprises an anti-HER2 domain (i.e., HER2-CARζ); and (ii) a CAR that comprises a VEGFR2 receptor extracellular domain (i.e., the CAR designated VEGFR2-CD28, VEGFR2-28TM-CD28, or VEGFR2). Expression of each CAR by the transfected T cells was confirmed using flow cytometry by measurement of reporter gene expression (i.e., tdTomato or GFP), as described above.

Expression of T cell activation markers CD69 and CD71 by T cells transfected with both HER2-CARζ and one of of the three VEGFR2 CAR constructs (i.e., the CAR construct designated VEGFR2-CD28, VEGFR2-28TM-CD28, or VEGFR2) was examined following stimulation of the T cells with HER2-Fc and either anti-VEGFR2 mAb or VEGF.

Then, dose-responsive enhancement of CD69 and CD71 expression was observed in T cells expressing GFP (i.e., T cells expressing a CAR construct comprising a VEGFR2 EC domain). Stimulation with VEGF also showed amplification of CD69 and CD71 expression in T cells expressing GFP (i.e., T cells expressing a CAR construct comprising a VEGFR2 EC domain). At the highest doses tested (1 ug/ml HER2-Fc/1 ug/ml anti-VEGFR2 or 1 ug/ml HER2-Fc/100 ng/ml anti-VEGF), robust increases in CD69 and CD71 expression were observed in T cells comprising the VEGFR2-28TM-CD28 construct compared to T cells comprising the control construct (i.e., the CAR construct designated VEGFR), therefore confirming VEGFR2 costimulation. A similar trend was observed when comparing CD69 and CD71 expression by T cells comprising the VEGFR2-CD28construct compared to T cells comprising the control construct (i.e., the CAR construct designated VEGFR).

This example demonstrates that functional CAR T cells can be generated that comprise two CARs, with the signaling domain present in a first CAR, and the costimulatory domains present in a second CAR. Such CAR T cells are useful in the treatment of diseases, e.g., cancer, where it is desirable to utilize a dual signaling approach that relies on the recognition of two separate antigens by the two CARs.

### 5.5. Example 5: Modified T Lymphocytes That Have Dual Antigen Specificity

This Example describes the generation of modified T lymphocytes comprising CARs that can be used in the dual signaling approach described in the present application. The modified T lymphocytes comprise a first chimeric antigen receptor that comprises an antigen binding domain specific to a tumor-specific antigen and a second chimeric antigen receptor that comprises an antigen binding domain specific to an antigen that is not a tumor-specific antigen, but that is associated with tumorigenesis. In this Example, the two CARs are introduced into the modified T cells using a single CAR construct, with the CARs separated by P2A, which allows for the expression of two, discrete CARs (at essentially equal amounts) from a single ORF.

Constructs comprising CARs are depicted in Figure 4. The first construct, "CAR1," comprises an anti-HER2 scFv, followed by a CD28 hinge, a CD28 transmembrane (TM) domain, a CD3 zeta chain, a T2A sequence, and a tdTomato reporter gene. "CAR2" comprises an anti-HER2 scFv, followed by a CD28 hinge, a CD28 transmembrane (TM) domain, a CD28 IC domain, a CD3 zeta chain, a T2A sequence, and a tdTomato reporter gene. "CAR3" comprises the human VEGFR2 extracellular (EC) domain, followed by a VEGFR2 TM domain, a P2A sequence, an anti-HER2 scFv, a CD28 hinge, a CD28 transmembrane (TM) domain, and a CD3 zeta chain. "CAR4"comprises the human VEGFR2 extracellular (EC) domain, followed by a CD28 transmembrane (TM) domain, a CD28 IC domain, an anti-HER2 scFv, a CD28 hinge, a CD28 transmembrane (TM) domain, and a CD3 zeta chain.

CAR1 represents a first generation anti-HER2 CAR that comprises a primary signaling domain (CD3 zeta chain), but lacks a costimulatory domain. CAR 2 represents a second generation anti-HER2 CAR that comprises both a primary signaling domain (CD3 zeta chain) and a costimulatory domain (CD28 IC domain). CAR3 is a dual CAR control construct; it comprises a HER2 primary signaling portion (HER2 scFV and CD3 zeta chain) and also comprises a VEGFR2 secondary signaling domain, but the secondary signaling domain lacks a costimulatory domain. CAR4 is a dual CAR construct; it comprises a HER2 primary signaling portion (HER2 scFV and CD3 zeta chain) and also comprises a VEGFR2 secondary signaling domain with a costimulatory domain (CD28 IC).

Pan T cells were isolated as described above, transfected with the CAR constructs (CAR1-CAR4) described above and analyzed 24-hours post-transduction. Expression of anti-HER2 was detected in T cells transfected with all of the CAR constructs. Expression of both anti-HER2 and VEGFR2 was detected in T cells transfected with CAR3 or CAR4. Thus, proper expression of the CAR constructs described above by T cells was confirmed.

Once expression of the CAR constructs was confirmed, T cells expressing the constructs were cultured with HER2-Fc (0.25 ug/ml or 1.0 ug/ml) - to induce stimulation the HER2-scFv containing constructs (primary signaling) - alone or in combination with either an anti-VEGFR2 antibody (0.25 ug/ml or 1.0 ug/ml) or VEGF (1, 10, or 100 ng/ml) - to induce stimulation of VEGFR2 containing constructs (costimulation). VEGFR2 activation was found not to alter surface marker expression T cell activation markers CD69 or CD71 (as assessed by flow cytometry) in T cells transfected with either CAR1 or CAR2 over stimulation observed with anti-HER2 activation alone.

In contrast, in T cells transfected with CAR4, stimulation with both HER2-Fc and anti-VEGFR2 resulted in enhanced CD69 expression compared with the CD69 expression in CAR4-expressing CAR T cells stimulated with HER2-Fc alone. This VEGFR2-mediated augmentation of CD69 expression up-regulation upon HER2-Fc stimulation is not observed in T cells transfected with the control dual stimulation CAR construct (CAR3) that lacks a costimulatory domain in the VEGFR2 CAR of the construct (i.e., CAR3). In particular, 73.6% and 72.9% of CAR4-expressing CAR T cells expressed CD69 when stimulated with doses of 0.25 ug/ml HER2-Fc/0.25 ug/ml anti-VEGFR2 and of 0.25 ug/ml HER2-Fc/1.0 ug/ml anti-VEGFR2, respectively, as compared to 33.4% of CD69⁺ CAR4-expressing CAR T cells stimulated with 0.25 µg/ml HER2-Fc alone; whereas only 6.13% and 3.69% of CAR3-expressing CAR T cells expressed CD69 when stimulated with HER2-Fc and anti-VEGFR2 at the same doses, respectively, as compared to 4.9% of CD69⁺ CAR3-expressing CAR T cells stimulated with 0.25 µg/ml HER2-Fc alone. A similar result was observed when CD71 expression was analyzed: 45.2% and 50.7% of CAR4-expressing CAR T cells expressed CD71 when stimulated with doses of 1.0 ug/ml HER2-Fc/0.25 ug/ml anti-VEGFR2 and of 1.0 ug/ml HER2-Fc/1.0 ug/ml anti-VEGFR2, respectively, as compared to 22.8% of CD71⁺ CAR4-expressing CAR T cells stimulated with 1.0 µg/ml HER2-Fc alone ; whereas only 7.80% and 7.89% of CAR3-expressing CAR T cells expressed CD71 when stimulated with HER2-Fc and anti-VEGFR2 at the same doses, respectively, as compared to 10.3% of CD71⁺ CAR3-expressing CAR T cells stimulated with 1.0 µg/ml HER2-Fc alone.

Likewise, in T cells transduced with CAR4, stimulation with both HER2-Fc and VEGF resulted in enhanced CD69 expression over levels of CD69 expression in T cells transduced with the control dual stimulation CAR construct (CAR3) that lacks a costimulatory domain in the VEGFR2 CAR of the construct (i.e., CAR3). In particular, 35.3%, 48.2%, and 48.5 % of CAR4-expressing CAR T cells expressed CD69 when stimulated with doses of 0.25 ug/ml HER2-Fc/1 ng/ml VEGF, 0.25 ug/ml HER2-Fc/10 ng/ml VEGF, and 0.25 ug/ml HER2-Fc/100 ng/ml VEGF, respectively, as compared to 33.4% of CD69⁺ CAR4-expressing CAR T cells when stimulated with 0.25 µg/ml HER2-Fc alone; whereas only 3.40%, 2.69%, and 2.55% of CAR3-expressing CAR T cells expressed CD69 when stimulated with HER2-Fc and VEGF at the same doses, respectively, as compared to 4.9% of CD69⁺ CAR3-expressing CAR T cells stimulated with 0.25 µg/ml HER2-Fc alone . In terms of CD71 expression, it was determined that 30.10%, 42.30%, and 47.30% of CAR4-expressing CAR T cells expressed CD71 when stimulated with doses of 1.0 ug/ml HER2-Fc/1 ng/ml VEGF, 1.0 ug/ml HER2-Fc/10 ng/ml VEGF, and 1.0 ug/ml HER2-Fc/100 ng/ml VEGF, respectively, as compared to 22.8% of CD71⁺ CAR4-expressing CAR T cells stimulated with 1.0 µg/ml HER2-Fc alone; whereas only 10.70%, 4.81%, and 7.33% of CAR3-expressing CAR T cells expressed CD69 when stimulated with HER2-Fc and VEGF at the same doses, respectively, as compared to 10.3% of CD71⁺ CAR3-expressing CAR T cells stimulated with 1.0 µg/ml HER2-Fc alone.

Granzyme B is an enzyme enzyme present in cytotoxic T lymphocyte granules. Granzyne B secretion by T cells transfected with either CAR1, CAR3, or CAR4 was assessed. T cells transfected with CAR4 expressed increased levels of granzyme B when stimulated with HER2-Fc and anti-VEGFR2 at doses of .25 ug/ml HER2-Fc/0.25 ug/ml anti-VEGFR2 and of 0.25 ug/ml HER2-Fc/1.0 ug/ml anti-VEGFR2 as compared to T cells transfected with either control CAR (CAR1 or CAR3). Likewise, T cells transfected with CAR4 expressed increased levels of granzyme B when stimulated with HER2-Fc and VEGF at doses of .25 ug/ml HER2-Fc/1ng/ml VEGF and of 0.25 ug/ml HER2-Fc/100 ng/ml VEGF as compared to T cells transfected with either control CAR (CAR1 or CAR3).

Viability of T cells transfected with CAR1, CAR3, or CAR4 following stimulation with HER2-Fc in combination with either an anti-VEGFR2 antibody or VEGF was assessed. Pan T cells were isolated as described. After 24 hours of culture, the T cells were stimulated with HER2-Fc (1.0 ug/ml) and either anti-VEGFR2 antibody (0.25 ug/ml or 1.0 ug/ml) or VEGF (1 ng/ml or 100 ng/ml) for 48 hours. After 13 total days of culture, viability of the T cells was determined. In each case, T cells transfected with the dual stimulation CAR (i.e., CAR4) showed increased viability over T cells transfected with the dual control CAR (i.e., CAR3) or the control CAR designated CAR1.

This example confirms the result of Example 4 - that functional CAR T cells can be generated that comprise two CARs, with the signaling domain present in a first CAR the costimulatory domains present in a second CAR - and further demonstrates that the two CAR constructs can be expressed in the CAR T cells as a single construct (e.g., can be the T cells can be transfected with a single CAR construct that comprises both of the CARs).

### EQUIVALENTS

The present disclosure is not to be limited in scope by the specific embodiments described herein. Indeed, various modifications of the subject matter provided herein, in addition to those described, will become apparent to those skilled in the art from the foregoing description. Such modifications are intended to fall within the scope of the appended claims.

Various publications, patents and patent applications are cited herein, the disclosures of which are incorporated by reference in their entireties.

### Also disclosed herein are inter alia the following items (said items are not claims):

1. A modified T lymphocyte comprising:
   a. a first polypeptide comprising a first extracellular antigen binding domain that binds a first antigen, and a first intracellular signaling domain, wherein said first polypeptide does not comprise a co-stimulatory domain; and
   b. a second polypeptide comprising a second extracellular antigen binding domain binding a second antigen, or a receptor that binds said second antigen; and a second intracellular signaling domain;
   wherein said modified lymphocyte becomes maximally cytotoxic only when said first signaling domain and said second signaling domain are both activated by said first antigen and said second antigen, respectively.
2. The modified T lymphocyte of item 1, wherein binding of said first antigen to said first antigen binding domain without binding of said second antigen to said second binding domain, or binding of said second antigen to said second antigen binding domain without binding of first second antigen to said first binding domain induces anergy of said modified T lymphocyte.
3. The modified T lymphocyte of item 1, wherein said first antigen binding domain and said second antigen binding domain are independently an antigen-binding portion of a receptor or an antigen-binding portion of an antibody.
4. The modified T lymphocyte of item 3, wherein either or both of said first antigen binding domain or said second antigen binding domain are scFv antibody fragments.
5. The modified T lymphocyte of item 1, wherein said first polypeptide additionally comprises a transmembrane domain.
6. The modified T lymphocyte of item 1, wherein said second polypeptide additionally comprises a transmembrane domain.
7. The modified T lymphocyte of any of items 1-6, wherein said first polypeptide or said second polypeptide comprises a T cell survival motif.
8. The modified T lymphocyte of item 1, wherein said first antigen is an antigen on a tumor cell.
9. The modified T lymphocyte of item 8, wherein said tumor cell is a cell in a solid tumor.
10. The modified T lymphocyte of item 8, wherein said antigen is a tumor-associated antigen or a tumor-specific antigen.
11. The modified T lymphocyte of item 10, wherein said tumor-associated antigen or tumor-specific antigen is Her2, prostate stem cell antigen (PSCA), PSMA, BCMA, alpha-fetoprotein (AFP), carcinoembryonic antigen (CEA), cancer antigen-125 (CA-125), CA19-9, calretinin, MUC-1, epithelial membrane protein (EMA), epithelial tumor antigen (ETA), tyrosinase, melanoma-associated antigen (MAGE), CD34, CD45, CD99, CD117, chromogranin, cytokeratin, desmin, glial fibrillary acidic protein (GFAP), gross cystic disease fluid protein (GCDFP-15), HMB-45 antigen, protein melan-A (melanoma antigen recognized by T lymphocytes; MART-1), myo-D1, muscle-specific actin (MSA), neurofilament, neuron-specific enolase (NSE), placental alkaline phosphatase, synaptophysis, thyroglobulin, thyroid transcription factor-1, the dimeric form of the pyruvate kinase isoenzyme type M2 (tumor M2-PK), CD19, CD22, CD27, CD30, CD70, GD2 (ganglioside G2), EGFRvIII (epidermal growth factor variant III), sperm protein 17 (Sp17), mesothelin, PAP (prostatic acid phosphatase), prostein, TARP (T cell receptor gamma alternate reading frame protein), Trp-p8, STEAP1 (six-transmembrane epithelial antigen of the prostate 1), an abnormal ras protein, or an abnormal p53 protein.
12. The modified T lymphocyte of item 1, wherein said first antigen is integrin αvβ3 (CD61), galactin, K-Ras (V-Ki-ras2 Kirsten rat sarcoma viral oncogene) or Ral-B.
13. The modified T lymphocyte of item 1, wherein said first intracellular signaling domain comprises a polypeptide sequence comprising an immunoreceptor tyrosine-based activation motif (ITAM).
14. The modified T lymphocyte of item 13, wherein said polypeptide sequence is a CD3ζ signaling domain.
15. The modified T lymphocyte of item 1, wherein said second antigen is a growth factor, cytokine, or interleukin.
16. The modified T lymphocyte of item 15, wherein said second antigen is a growth factor, cytokine, or interleukin associated with angiogenesis or vasculogenesis.
17. The modified T lymphocyte of item 15, wherein said second antigen is vascular endothelial growth factor (VEGF), basic fibroblast growth factor (bFGF), platelet-derived growth factor (PDGF), hepatocyte growth factor (HGF), insulin-like growth factor (IGF), or interleukin-8 (IL-8).
18. The modified T lymphocyte of item 1, wherein signal transduction by said second polypeptide is induced by activation of a hypoxia-associated factor.
19. The modified T lymphocyte of item 18, wherein said hypoxia-associated factor is hypoxia-inducible factor-la (HIF-1α), HIF-1β, HIF-2α, HIF-2β, HIF-3α, or HIP-3β.
20. The modified T lymphocyte of item 1, wherein said second antigen is an interleukin.
21. The modified T lymphocyte of item 1, wherein said second antigen is a damage associated molecular pattern molecule (DAMP; also known as an alarmin).
22. The modified T lymphocyte of item 21, wherein said DAMP is a heat shock protein, chromatin-associated protein high mobility group box 1 (HMGB1), S100A8 (MRP8, calgranulin A), S100A9 (MRP14, calgranulin B), serum amyloid A (SAA), deoxyribonucleic acid, adenosine triphosphate, uric acid, or heparin sulfate.
23. The modified T lymphocyte of item 1, wherein said second antigen is an antigen on an antibody that binds to an antigen presented by a tumor cell.
24. The modified T lymphocyte of any of items 1-23, wherein said second polypeptide comprises one or more co-stimulatory domains.
25. The modified T lymphocyte of item 24, wherein said one or more co-stimulatory domains comprises one or more of a co-stimulatory CD27 polypeptide sequence, a costimulatory CD28 polypeptide sequence, a co-stimulatory OX40 (CD134) polypeptide sequence, a co-stimulatory 4-1BB (CD137) polypeptide sequence, or a co-stimulatory inducible T-cell costimulatory (ICOS) polypeptide sequence.
26. The modified T lymphocyte of item 1, wherein said first polypeptide comprises an extracellular tumor antigen-binding domain and a CD3ζ signaling domain, and wherein said second polypeptide comprises an antigen-binding domain wherein said antigen is an angiogenic or vasculogenic factor, and one or more co-stimulatory molecule signaling motifs.
27. The modified T lymphocyte of item 26, wherein said angiogenic factor is VEGF.
28. The modified T lymphocyte of item 26, wherein said one or more co-stimulatory molecule signaling motifs comprise co-stimulatory signaling motifs from each of CD27, CD28, OX40, ICOS, and 4-1BB.
29. The modified T lymphocyte of item 28, wherein said first polypeptide comprises an extracellular tumor antigen-binding domain and a CD3ζ signaling domain, and wherein said second polypeptide comprises an antigen-binding domain wherein said antigen is VEGF, and costimulatory signaling motifs from each of CD27, CD28, OX40, ICOS, and 4-1BB.
30. The modified T lymphocyte of item 1, wherein said first polypeptide or said second polypeptide comprises a T cell survival motif.
31. The modified T lymphocyte of item 29, wherein said first polypeptide or said second polypeptide comprises a T cell survival motif.
32. The modified T lymphocyte of item 31, wherein said T cell survival motif is, or is derived from, an intracellular signaling domain of IL-7 receptor (IL-7R), an intracellular signaling domain of IL-12 receptor, an intracellular signaling domain of IL-15 receptor, an intracellular signaling domain of IL-21 receptor, or an intracellular signaling domain of transforming growth factor β (TGFβ) receptor.
33. The modified T lymphocyte of item 26, wherein said first polypeptide comprises an extracellular tumor antigen-binding domain and a CD3ζ signaling domain, and wherein said second polypeptide comprises an antigen-binding domain wherein said antigen is VEGF, an IL-7 receptor intracellular T cell survival motif, and co-stimulatory signaling motifs from each of CD27, CD28, OX40, ICOS, and 4-1BB.
34. The modified T lymphocyte of item 1, wherein said first antigen is a tumor-specific antigen or a tumor-associated antigen, and said first intracellular signaling domain comprises a CD3ζ signaling domain; and wherein said second polypeptide comprises an antigen-binding domain that binds said second antigen, and co-stimulatory signaling motifs from each of CD27, CD28, OX40, ICOS, and 4-1BB.
35. The modified T lymphocyte of item 34, wherein said second polypeptide further comprises an intracellular T cell survival motif.
36. The modified T lymphocyte of item 35, wherein said T cell survival motif is, or is derived from, an intracellular signaling domain of IL-7 receptor (IL-7R), an intracellular signaling domain of IL-12 receptor, an intracellular signaling domain of IL-15 receptor, an intracellular signaling domain of IL-21 receptor, or an intracellular signaling domain of transforming growth factor β (TGFβ) receptor.
37. The modified T lymphocyte of item 36, wherein said T cell survival motif is, or is derived from, an intracellular signaling domain of IL-7R.
38. The modified T lymphocyte of item 34, wherein said second antigen is VEGF.
39. The modified T lymphocyte of item 34, wherein said second antigen is IL-4.
40. The modified T lymphocyte of item 36, wherein said second antigen is VEGF.
41. The modified T lymphocyte of item 36, wherein said second antigen is IL-4.
42. The modified T lymphocyte of item 1, wherein said first polypeptide comprises an extracellular tumor antigen-binding domain and a CD3ζ signaling domain, and wherein said second polypeptide comprises an antigen-binding domain wherein said antigen is an interleukin, and one or more co-stimulatory molecule signaling motifs.
43. The modified T lymphocyte of item 42, wherein said interleukin is IL-4.
44. The modified T lymphocyte of item 43, wherein said second antigen binding domain is an IL-4-binding portion of an IL-4 receptor.
45. The modified T lymphocyte of item 44, wherein said first polypeptide or said second polypeptide comprises a T cell survival motif.
46. The modified T lymphocyte of item 45, wherein said T cell survival motif is, or is derived from, an intracellular signaling domain of IL-7 receptor (IL-7R), an intracellular signaling domain of IL-12 receptor, an intracellular signaling domain of IL-15 receptor, an intracellular signaling domain of IL-21 receptor, or an intracellular signaling domain of transforming growth factor β (TGFβ) receptor.
47. The modified T lymphocyte of item 1, wherein said first polypeptide comprises an extracellular tumor antigen-binding domain and a CD3ζ signaling domain, and wherein said second polypeptide comprises an IL-4-binding portion of an IL-4 receptor, an IL-7 receptor intracellular T cell survival motif, and co-stimulatory signaling motifs from each of CD27, CD28, OX40, ICOS, and 4-1BB.
48. A modified T lymphocyte comprising:
   a. a first polypeptide comprising a first extracellular antigen binding domain that binds a first antigen, and a first intracellular signaling domain; and
   b. a second polypeptide comprising a second extracellular antigen binding domain binding a second antigen, or a receptor that binds said second antigen; and a second intracellular signaling domain, wherein said second polypeptide does not comprise a co-stimulatory domain;
   wherein said modified lymphocyte becomes maximally cytotoxic only when said first signaling domain and said second signaling domain are both activated by said first antigen and said second antigen, respectively.
49. The modified T lymphocyte of item 48, wherein binding of said first antigen to said first antigen binding domain without binding of said second antigen to said second binding domain, or binding of said second antigen to said second antigen binding domain without binding of first second antigen to said first binding domain induces anergy of said modified T lymphocyte.
50. The modified T lymphocyte of item 48, wherein said first antigen-binding domain and said antigen-binding domain are independently an antigen-binding portion of a receptor or an antigen-binding portion of an antibody.
51. The modified T lymphocyte of item 50, wherein either or both of said first antigen binding domain or said second antigen binding domain are scFv antibody fragments.
52. The modified T lymphocyte of item 48, wherein said first polypeptide additionally comprises a transmembrane domain.
53. The modified T lymphocyte of item 48, wherein said second polypeptide additionally comprises a transmembrane domain.
54. The modified T lymphocyte of any of items 48-53, wherein said first polypeptide or said second polypeptide comprises a T cell survival motif.
55. The modified T lymphocyte of item 43, wherein said first antigen is an antigen on a tumor cell.
56. The modified T lymphocyte of item 55, wherein said tumor cell is a cell in a solid tumor.
57. The modified T lymphocyte of item 55, wherein said first antigen is a tumor-associated antigen or a tumor-specific antigen.
58. The modified T lymphocyte of item 57, wherein said tumor-associated antigen or tumor-specific antigen is Her2, prostate stem cell antigen (PSCA), PSMA, BCMA, alpha-fetoprotein (AFP), carcinoembryonic antigen (CEA), cancer antigen-125 (CA-125), CA19-9, calretinin, MUC-1, epithelial membrane protein (EMA), epithelial tumor antigen (ETA), tyrosinase, melanoma-associated antigen (MAGE), CD34, CD45, CD99, CD117, chromogranin, cytokeratin, desmin, glial fibrillary acidic protein (GFAP), gross cystic disease fluid protein (GCDFP-15), HMB-45 antigen, protein melan-A (melanoma antigen recognized by T lymphocytes; MART-1), myo-D1, muscle-specific actin (MSA), neurofilament, neuron-specific enolase (NSE), placental alkaline phosphatase, synaptophysis, thyroglobulin, thyroid transcription factor-1, the dimeric form of the pyruvate kinase isoenzyme type M2 (tumor M2-PK), CD19, CD22, CD27, CD30, CD70, GD2 (ganglioside G2), EGFRvIII (epidermal growth factor variant III), sperm protein 17 (Sp17), mesothelin, PAP (prostatic acid phosphatase), prostein, TARP (T cell receptor gamma alternate reading frame protein), Trp-p8, STEAP1 (six-transmembrane epithelial antigen of the prostate 1), an abnormal ras protein, or an abnormal p53 protein.
59. The modified T lymphocyte of item 48, wherein said first antigen is integrin αvβ3 (CD61), galactin, K-Ras (V-Ki-ras2 Kirsten rat sarcoma viral oncogene) or Ral-B.
60. The modified T lymphocyte of item 48, wherein said second intracellular signaling domain comprises a polypeptide sequence comprising an immunoreceptor tyrosine-based activation motif (ITAM).
61. The modified T lymphocyte of item 60, wherein said polypeptide sequence is a CD3ζ signaling domain.
62. The modified T lymphocyte of item 48, wherein said second antigen is a growth factor, cytokine, or interleukin.
63. The modified T lymphocyte of item 62, wherein said second antigen is a growth factor, cytokine, or interleukin associated with angiogenesis or vasculogenesis.
64. The modified T lymphocyte of item 62, wherein said second antigen is vascular endothelial growth factor (VEGF), basic fibroblast growth factor (bFGF), platelet-derived growth factor (PDGF), hepatocyte growth factor (HGF), insulin-like growth factor (IGF), or interleukin-8 (IL-8).
65. The modified T lymphocyte of item 48, wherein signal transduction by said second chimeric receptor is induced by activation of a hypoxia-associated factor.
66. The modified T lymphocyte of item 65, wherein said hypoxia-associated factor is hypoxia-inducible factor-la (HIF-1α), HIF-1β, HIF-2α, HIF-2β, HIF-3α, or HIP-3β.
67. The modified T lymphocyte of item 48, wherein said second antigen is an interleukin.
68. The modified T lymphocyte of item 48, wherein said second antigen is a DAMP.
69. The modified T lymphocyte of item 68, wherein said DAMP is a heat shock protein, chromatin-associated protein high mobility group box 1 (HMGB1), S100A8 (MRP8, calgranulin A), S100A9 (MRP14, calgranulin B), serum amyloid A (SAA), deoxyribonucleic acid, adenosine triphosphate, uric acid, or heparin sulfate.
70. The modified T lymphocyte of item 48, wherein said second antigen is an antigen on an antibody that binds to an antigen presented by a tumor cell.
71. The modified T lymphocyte of any of items 48-70, wherein said first polypeptide comprises one or more co-stimulatory domains.
72. The modified T lymphocyte of item 71 or item 72, wherein said one or more co-stimulatory domains comprises one or more of a co-stimulatory CD27 polypeptide sequence, a co-stimulatory CD28 polypeptide sequence, a co-stimulatory OX40 (CD134) polypeptide sequence, a co-stimulatory 4-1BB (CD137) polypeptide sequence, or a co-stimulatory inducible T-cell costimulatory (ICOS) polypeptide sequence.
73. The modified T lymphocyte of item 48, wherein said first polypeptide or said second polypeptide comprises a T cell survival motif.
74. The modified T lymphocyte of item 73, wherein said T cell survival motif is, or is derived from, an intracellular signaling domain of IL-7 receptor (IL-7R), an intracellular signaling domain of IL-12 receptor, an intracellular signaling domain of IL-15 receptor, an intracellular signaling domain of IL-21 receptor, or an intracellular signaling domain of transforming growth factor β (TGFβ) receptor.
75. A modified T lymphocyte comprising:
   a. a first polypeptide comprising a first extracellular antigen binding domain that binds a first antigen, an intracellular signaling domain, and one or more costimulatory motifs; and
   b. a second polypeptide comprising a second extracellular antigen binding domain that binds a second antigen, and a T cell survival motif, wherein said second polypeptide does not comprise an intracellular signaling domain or a co-stimulatory motif;
   wherein said modified lymphocyte survives only when said intracellular signaling domain and said T cell survival motif are both activated by said first antigen and said second antigen, respectively.
76. The modified T lymphocyte of item 75, wherein said T cell survival motif is an IL-7 receptor intracellular T cell survival motif.
77. The modified T lymphocyte of item 75, wherein said intracellular signaling domain is or comprises a CD3ζ signaling domain.
78. The modified T lymphocyte of item 75, wherein said one or more co-stimulatory motifs comprise one or more of a co-stimulatory CD27 polypeptide sequence, a co-stimulatory CD28 polypeptide sequence, a co-stimulatory OX40 (CD134) polypeptide sequence, a costimulatory 4-1BB (CD137) polypeptide sequence, or a co-stimulatory inducible T-cell costimulatory (ICOS) polypeptide sequence.
79. The modified T lymphocyte of item 75, wherein said first antigen is an antigen on a tumor cell.
80. The modified T lymphocyte of item 79, wherein said tumor cell is a cell in a solid tumor.
81. The modified T lymphocyte of item 79, wherein said antigen is a tumor-associated antigen or a tumor-specific antigen.
82. The modified T lymphocyte of item 81, wherein said tumor-associated antigen or tumor-specific antigen is Her2, prostate stem cell antigen (PSCA), alpha-fetoprotein (AFP), carcinoembryonic antigen (CEA), cancer antigen-125 (CA-125), CA19-9, calretinin, MUC-1, epithelial membrane protein (EMA), epithelial tumor antigen (ETA), tyrosinase, melanoma-associated antigen (MAGE), CD34, CD45, CD99, CD117, chromogranin, cytokeratin, desmin, glial fibrillary acidic protein (GFAP), gross cystic disease fluid protein (GCDFP-15), HMB-45 antigen, protein melan-A (melanoma antigen recognized by T lymphocytes; MART-1), myo-D1, muscle-specific actin (MSA), neurofilament, neuron-specific enolase (NSE), placental alkaline phosphatase, synaptophysis, thyroglobulin, thyroid transcription factor-1, the dimeric form of the pyruvate kinase isoenzyme type M2 (tumor M2-PK), CD19, CD22, CD27, CD30, CD70, GD2 (ganglioside G2), EGFRvIII (epidermal growth factor variant III), sperm protein 17 (Sp17), mesothelin, PAP (prostatic acid phosphatase), prostein, TARP (T cell receptor gamma alternate reading frame protein), Trp-p8, STEAP1 (six-transmembrane epithelial antigen of the prostate 1), an abnormal ras protein, or an abnormal p53 protein.
83. The modified T lymphocyte of item 75, wherein said first antigen is integrin αvβ3 (CD61), galactin, K-Ras (V-Ki-ras2 Kirsten rat sarcoma viral oncogene) or Ral-B.
84. The modified T lymphocyte of item 75, wherein said second antigen-binding domain is an IL-4-binding portion of an IL-4 receptor.
85. The modified T lymphocyte of item 75, wherein said second antigen is a growth factor, cytokine, or interleukin.
86. The modified T lymphocyte of item 83, wherein said second antigen is a growth factor, cytokine, or interleukin associated with angiogenesis or vasculogenesis.
87. The modified T lymphocyte of item 86, wherein said second antigen is vascular endothelial growth factor (VEGF), basic fibroblast growth factor (bFGF), platelet-derived growth factor (PDGF), hepatocyte growth factor (HGF), insulin-like growth factor (IGF), or interleukin-8 (IL-8).
88. The modified T lymphocyte of item 75, wherein signal transduction by said second chimeric receptor is induced by activation of a hypoxia-associated factor.
89. The modified T lymphocyte of item 88, wherein said hypoxia-associated factor is hypoxia-inducible factor-la (HIF-1α), HIF-1β, HIF-2α, HIF-2β, HIF-3α, or HIP-3β.
90. The modified T lymphocyte of item 75, wherein said second antigen is a damage associated molecular pattern molecule (DAMP; also known as an alarmin).
91. The modified T lymphocyte of item 90, wherein said DAMP is a heat shock protein, chromatin-associated protein high mobility group box 1 (HMGB1), S100A8 (MRP8, calgranulin A), S100A9 (MRP14, calgranulin B), serum amyloid A (SAA), deoxyribonucleic acid, adenosine triphosphate, uric acid, or heparin sulfate.
92. The modified T lymphocyte of item 75, wherein said second antigen is an antigen on an antibody that binds to an antigen presented by a tumor cell.
93. A modified T lymphocyte comprising:
   a. a first polypeptide comprising a first extracellular antigen binding domain that binds a first antigen, and a T cell survival motif, wherein said first polypeptide does not comprise an intracellular signaling domain or a co-stimulatory motif; and
   b. a second polypeptide comprising a second extracellular antigen binding domain that binds a second antigen, an intracellular signaling domain and one or more co-stimulatory motifs;
   wherein said modified lymphocyte survives only when said T cell survival motif and said intracellular signaling domain are both activated by said first antigen and said second antigen, respectively.
94. The modified T lymphocyte of item 93, wherein said T cell survival motif is an IL-7 receptor intracellular T cell survival motif.
95. The modified T lymphocyte of item 93, wherein said intracellular signaling domain is a CD3ζ signaling domain.
96. The modified T lymphocyte of item 93, wherein said one or more co-stimulatory motifs comprise one or more of a co-stimulatory CD27 polypeptide sequence, a co-stimulatory CD28 polypeptide sequence, a co-stimulatory OX40 (CD134) polypeptide sequence, a costimulatory 4-1BB (CD137) polypeptide sequence, or a co-stimulatory inducible T-cell costimulatory (ICOS) polypeptide sequence.
97. The modified T lymphocyte of item 93, wherein said first antigen is an antigen on a tumor cell.
98. The modified T lymphocyte of item 97, wherein said tumor cell is a cell in a solid tumor.
99. The modified T lymphocyte of item 97, wherein said antigen is a tumor-associated antigen or a tumor-specific antigen.
100. The modified T lymphocyte of item 99, wherein said tumor-associated antigen or tumor-specific antigen is Her2, prostate stem cell antigen (PSCA), alpha-fetoprotein (AFP), carcinoembryonic antigen (CEA), cancer antigen-125 (CA-125), CA19-9, calretinin, MUC-1, epithelial membrane protein (EMA), epithelial tumor antigen (ETA), tyrosinase, melanoma-associated antigen (MAGE), CD34, CD45, CD99, CD117, chromogranin, cytokeratin, desmin, glial fibrillary acidic protein (GFAP), gross cystic disease fluid protein (GCDFP-15), HMB-45 antigen, protein melan-A (melanoma antigen recognized by T lymphocytes; MART-1), myo-D1, muscle-specific actin (MSA), neurofilament, neuron-specific enolase (NSE), placental alkaline phosphatase, synaptophysis, thyroglobulin, thyroid transcription factor-1, the dimeric form of the pyruvate kinase isoenzyme type M2 (tumor M2-PK), CD19, CD22, CD27, CD30, CD70, GD2 (ganglioside G2), EGFRvIII (epidermal growth factor variant III), sperm protein 17 (Sp17), mesothelin, PAP (prostatic acid phosphatase), prostein, TARP (T cell receptor gamma alternate reading frame protein), Trp-p8, STEAP1 (six-transmembrane epithelial antigen of the prostate 1), an abnormal ras protein, or an abnormal p53 protein.
101. The modified T lymphocyte of item 93, wherein said first antigen is integrin αvβ3 (CD61), galactin, K-Ras (V-Ki-ras2 Kirsten rat sarcoma viral oncogene) or Ral-B.
102. The modified T lymphocyte of item 93, wherein said second antigen-binding domain is an IL-4-binding portion of an IL-4 receptor.
103. The modified T lymphocyte of item 93, wherein said second antigen is a growth factor, cytokine, or interleukin.
104. The modified T lymphocyte of item 93, wherein said second antigen is a growth factor, cytokine, or interleukin associated with angiogenesis or vasculogenesis.
105. The modified T lymphocyte of item 104, wherein said second antigen is vascular endothelial growth factor (VEGF), basic fibroblast growth factor (bFGF), platelet-derived growth factor (PDGF), hepatocyte growth factor (HGF), insulin-like growth factor (IGF), or interleukin-8 (IL-8).
106. The modified T lymphocyte of item 93, wherein signal transduction by said second chimeric receptor is induced by activation of a hypoxia-associated factor.
107. The modified T lymphocyte of item 106, wherein said hypoxia-associated factor is hypoxia-inducible factor-la (HIF-1α), HIF-1β, HIF-2α, HIF-2β, HIF-3α, or HIP-3β.
108. The modified T lymphocyte of item 93, wherein said second antigen is a damage associated molecular pattern molecule (DAMP; also known as an alarmin).
109. The modified T lymphocyte of item 108, wherein said DAMP is a heat shock protein, chromatin-associated protein high mobility group box 1 (HMGB1), S100A8 (MRP8, calgranulin A), S100A9 (MRP14, calgranulin B), serum amyloid A (SAA), deoxyribonucleic acid, adenosine triphosphate, uric acid, or heparin sulfate.
110. The modified T lymphocyte of item 93, wherein said second antigen is an antigen on an antibody that binds to an antigen presented by a tumor cell.

## Claims

1. A modified T lymphocyte comprising:
a first polypeptide comprising a first extracellular antigen binding domain that binds a first antigen, and a first intracellular signaling domain, wherein said first polypeptide does not comprise a co-stimulatory domain; and
a second polypeptide comprising a second extracellular antigen binding domain binding a second antigen, or a receptor that binds said second antigen; and a second intracellular signaling domain, wherein said second polypeptide comprises one or
more co-stimulatory domains, wherein said second antigen is:
(i) a growth factor, preferably vascular endothelial growth factor (VEGF), basic fibroblast growth factor (bFGF), platelet-derived growth factor (PDGF), hepatocyte growth factor (HGF), insulin-like growth factor (IGF);
(ii) a cytokine;
(iii) an interleukin, preferably an interleukin associated with angiogenesis or vasculogenesis;
(iv) interleukin-8 (IL-8), IL-4;
(v) a hypoxia-associated factor, preferably hypoxia-inducible factor-la (HIF-1α), HIF-1β, HIF-2α, HIF-2β, HIF-3α, or HIF-3β;
(vi) a damage associated molecular pattern molecule (DAMP; also known as an alarmin), preferably a heat shock protein, chromatin-associated protein high mobility group box 1 (HMGB1), S100A8 (MRP8, calgranulin A), S100A9 (MRP14, calgranulin B), serum amyloid A (SAA), deoxyribonucleic acid, adenosine triphosphate, uric acid, or heparin sulfate; or
(vii) an antigen on an antibody that binds to an antigen presented by a tumor cell, wherein said modified lymphocyte becomes maximally cytotoxic only when said first signaling domain and said second signaling domain are both activated by said first antigen and said second antigen, respectively.

2. The modified T lymphocyte of claim 1, wherein said first antigen binding domain and said second antigen binding domain are independently an antigen-binding portion of a receptor or an antigen-binding portion of an antibody, preferably an scFv antibody fragment.

3. The modified T lymphocyte of claim 1 or 2, wherein said first intracellular signaling domain comprises a polypeptide sequence comprising an immunoreceptor tyrosine-based activation motif (ITAM), wherein said polypeptide sequence is preferably a CD3ζ signaling domain.

4. The modified T lymphocyte of any one of claims 1 to 3, wherein said first antigen is:
(i) an antigen on a tumor cell, wherein said tumor cell is preferably a cell in a solid tumor;
(ii) a tumor-associated antigen or a tumor-specific antigen, wherein said tumor-associated antigen or tumor-specific antigen is preferably Her2, prostate stem cell antigen (PSCA), PSMA, BCMA, alpha-fetoprotein (AFP), carcinoembryonic antigen (CEA), cancer antigen-125 (CA-125), CA19-9, calretinin, MUC-1, epithelial membrane protein (EMA), epithelial tumor antigen (ETA), tyrosinase, melanoma-associated antigen (MAGE), CD34, CD45, CD99, CD117, chromogranin, cytokeratin, desmin, glial fibrillary acidic protein (GFAP), gross cystic disease fluid protein (GCDFP-15), HMB-45 antigen, protein melan-A (melanoma antigen recognized by T lymphocytes; MART-1), myo-D1, muscle-specific actin (MSA), neurofilament, neuron-specific enolase (NSE), placental alkaline phosphatase, synaptophysis, thyroglobulin, thyroid transcription factor-1, the dimeric form of the pyruvate kinase isoenzyme type M2 (tumor M2-PK), CD19, CD22, CD27, CD30, CD70, GD2 (ganglioside G2), EGFRvIII (epidermal growth factor variant III), sperm protein 17 (Sp17), mesothelin, PAP (prostatic acid phosphatase), prostein, TARP (T cell receptor gamma alternate reading frame protein), Trp-p8, STEAP1 (six-transmembrane epithelial antigen of the prostate 1), an abnormal ras protein, or an abnormal p53 protein; or
(iii) integrin αvβ3 (CD61), galactin, K-Ras (V-Ki-ras2 Kirsten rat sarcoma viral oncogene) or Ral-B.

5. The modified T lymphocyte of any one of claims 1 to 4, wherein said one or more costimulatory domains preferably comprises one or more of a co-stimulatory CD27 polypeptide sequence, a co-stimulatory CD28 polypeptide sequence, a co-stimulatory OX40 (CD134) polypeptide sequence, a co-stimulatory 4-1BB (CD137) polypeptide sequence, or a co-stimulatory inducible T-cell costimulatory (ICOS) polypeptide sequence.

6. The modified T lymphocyte of claim 5, wherein said first polypeptide comprises an extracellular tumor antigen-binding domain and a CD3ζ signaling domain, and wherein said second polypeptide comprises an antigen-binding domain that binds said second antigen, wherein said second antigen is an angiogenic or vasculogenic factor, preferably VEGF; and one or more co-stimulatory domains, wherein said one or more co-stimulatory domains preferably comprise co-stimulatory signaling motifs from each of CD27, CD28, OX40, ICOS, and 4-1BB.

7. The modified T lymphocyte of any one of claims 1 to 6, wherein said first polypeptide and/or said second polypeptide comprises an intracellular signaling domain of IL-7 receptor (IL-7R), an intracellular signaling domain of IL-12 receptor, an intracellular signaling domain of IL-15 receptor, an intracellular signaling domain of IL-21 receptor, or an intracellular signaling domain of transforming growth factor β (TGFβ) receptor.

8. The modified T lymphocyte of claim 7, wherein said first polypeptide comprises an extracellular tumor antigen-binding domain and a CD3ζ signaling domain, and wherein said second polypeptide comprises an antigen-binding domain that binds said second antigen, wherein said second antigen is preferably VEGF or IL-4; an intracellular signaling domain of IL-7 receptor; and one or more co-stimulatory domains, wherein said one or more co-stimulatory domains comprise co-stimulatory signaling motifs from each of CD27, CD28, OX40, ICOS, and 4-1BB.

9. A modified T lymphocyte comprising:
a first polypeptide comprising a first extracellular antigen binding domain that binds a first antigen, and a first intracellular signaling domain, wherein said first polypeptide comprises one or more co-stimulatory domains; and
a second polypeptide comprising a second extracellular antigen binding domain binding a second antigen, or a receptor that binds said second antigen; and a second intracellular signaling domain, wherein said second polypeptide does not comprise a co-stimulatory domain, wherein said second antigen is:
(i) a growth factor, preferably vascular endothelial growth factor (VEGF), basic fibroblast growth factor (bFGF), platelet-derived growth factor (PDGF), hepatocyte growth factor (HGF), insulin-like growth factor (IGF);
(ii) a cytokine;
(iii) an interleukin, preferably an interleukin associated with angiogenesis or vasculogenesis;
(iv) interleukin-8 (IL-8), IL-4;
(v) a hypoxia-associated factor, preferably hypoxia-inducible factor-la (HIF-1α), HIF-1β, HIF-2α, HIF-2β, HIF-3α, or HIF-3β;
(vi) a damage associated molecular pattern molecule (DAMP; also known as an alarmin), preferably a heat shock protein, chromatin-associated protein high mobility group box 1 (HMGB 1), S 100A8 (MRP8, calgranulin A), S100A9 (MRP14, calgranulin B), serum amyloid A (SAA), deoxyribonucleic acid, adenosine triphosphate, uric acid, or heparin sulfate; or
(vii) an antigen on an antibody that binds to an antigen presented by a tumor cell,
wherein said modified lymphocyte becomes maximally cytotoxic only when said first signaling domain and said second signaling domain are both activated by said first antigen and said second antigen, respectively.

10. The modified T lymphocyte of claim 9, wherein said first antigen binding domain and said second antigen binding domain are independently an antigen-binding portion of a receptor or an antigen-binding portion of an antibody, preferably an scFv antibody fragment.

11. The modified T lymphocyte of claim 9 or 10, wherein said second intracellular signaling domain comprises a polypeptide sequence comprising an immunoreceptor tyrosine-based activation motif (ITAM), wherein said polypeptide sequence is preferably a CD3ζ signaling domain.

12. The modified T lymphocyte of any one of claims 9 to 11, wherein said first antigen is:
(i) an antigen on a tumor cell, wherein said tumor cell is preferably a cell in a solid tumor;
(ii) a tumor-associated antigen or a tumor-specific antigen, wherein said tumor-associated antigen or tumor-specific antigen is preferably Her2, prostate stem cell antigen (PSCA), PSMA, BCMA, alpha-fetoprotein (AFP), carcinoembryonic antigen (CEA), cancer antigen-125 (CA-125), CA19-9, calretinin, MUC-1, epithelial membrane protein (EMA), epithelial tumor antigen (ETA), tyrosinase, melanoma-associated antigen (MAGE), CD34, CD45, CD99, CD117, chromogranin, cytokeratin, desmin, glial fibrillary acidic protein (GFAP), gross cystic disease fluid protein (GCDFP-15), HMB-45 antigen, protein melan-A (melanoma antigen recognized by T lymphocytes; MART-1), myo-D1, muscle-specific actin (MSA), neurofilament, neuron-specific enolase (NSE), placental alkaline phosphatase, synaptophysis, thyroglobulin, thyroid transcription factor-1, the dimeric form of the pyruvate kinase isoenzyme type M2 (tumor M2-PK), CD19, CD22, CD27, CD30, CD70, GD2 (ganglioside G2), EGFRvIII (epidermal growth factor variant III), sperm protein 17 (Sp17), mesothelin, PAP (prostatic acid phosphatase), prostein, TARP (T cell receptor gamma alternate reading frame protein), Trp-p8, STEAP1 (six-transmembrane epithelial antigen of the prostate 1), an abnormal ras protein, or an abnormal p53 protein; or
(iii) integrin αvβ3 (CD61), galactin, K-Ras (V-Ki-ras2 Kirsten rat sarcoma viral oncogene) or Ral-B.

13. The modified T lymphocyte of any one of claims 9 to 12, wherein said one or more co-stimulatory domains preferably comprises one or more of a co-stimulatory CD27 polypeptide sequence, a co-stimulatory CD28 polypeptide sequence, a co-stimulatory OX40 (CD134) polypeptide sequence, a co-stimulatory 4-1BB (CD137) polypeptide sequence, or a co-stimulatory inducible T-cell costimulatory (ICOS) polypeptide sequence.

14. The modified T lymphocyte of claim 13, wherein said one or more co-stimulatory domains comprise co-stimulatory signaling motifs from each of CD27, CD28, OX40, ICOS, and 4-1BB; and wherein said second polypeptide comprises an extracellular tumor antigen-binding domain and a CD3ζ signaling domain, wherein said second antigen is an angiogenic or vasculogenic factor, preferably VEGF or IL-4.

15. The modified T lymphocyte of any one of claims 9 to 14, wherein said first polypeptide and/or said second polypeptide comprises an intracellular signaling domain of IL-7 receptor (IL-7R), an intracellular signaling domain of IL-12 receptor, an intracellular signaling domain of IL-15 receptor, an intracellular signaling domain of IL-21 receptor, or an intracellular signaling domain of transforming growth factor β (TGFβ) receptor.
